# EUROPEAN PATENT SPECIFICATION

(11) **EP 3 193 829 B1**
(45) Date of publication and mention of the grant of the patent: **08.12.2021**
(21) Application number: 15771549.1
(22) Date of filing: 18.09.2015
(51) Int. Cl.: A61K 9/00, A61K 9/16, A61F 9/00, A61K 31/436, A61K 31/56, A61K 31/573, A61K 31/7052, A61K 31/7105, A61K 47/32, A61K 47/34, A61P 9/00, A61P 25/00, A61P 27/02, A61P 27/06, A61P 29/00, A61P 31/00, A61P 43/00, A61M 5/32

(54) **OPHTHALMIC DRUG COMPOSITIONS**
OPHTHALMISCHE ARZNEIMITTELZUSAMMENSETZUNGEN
COMPOSITIONS MÉDICAMENTEUSES OPHTALMIQUES

(30) Priority: 19.09.2014 US 201462052969 P
(43) Date of publication of application: 26.07.2017
(73) Proprietor: Oxular Limited, Oxford OX4 4GA (GB)
(72) Inventor: YAMAMOTO, Ronald K., San Francisco, CA 94117 (US); CONSTON, Stanley R., San Carlos, CA 94070 (US); NGUYEN, Tien, Daly City, CA 94014 (US)
(74) Representative: Bassil, Nicholas Charles
(86) International application number: PCT/EP2015/071522
(87) International publication number: WO 2016/042163

(56) References cited:
- US-A1- 2005 048 099
- US-A1- 2008 131 484
- US-A1- 2011 238 075
- US-A1- 2013 216 623
- US-A1- 2013 289 467
- HERRERO-VANRELL ROCÍO ET AL: "The potential of using biodegradable microspheres in retinal diseases and other intraocular pathologies", PROGRESS IN RETINAL AND EYE RESEARCH, OXFORD, GB, vol. 42, 10 May 2014 (2014-05-10), pages 27-43, XP029046400, ISSN: 1350-9462, DOI: 10.1016/J.PRETEYERES.2014.04.002
- KOMPELLA UDAY B ET AL: "Nanomedicines for back of the eye drug delivery, gene delivery, and imaging", PROGRESS IN RETINAL AND EYE RESEARCH, vol. 36, 17 April 2013 (2013-04-17), pages 172-198, XP028703477, ISSN: 1350-9462, DOI: 10.1016/J.PRETEYERES.2013.04.001

## Description

### Background of Invention:

Due to the unique anatomy and physiology of the eye, multiple barriers exist that prevent significant transport of drugs to ocular tissues. The blood vessels of the eye have restricted permeability due to the blood-ocular barriers that regulate intraocular fluid. Due to these blood- ocular barriers, systemically administered drugs do not reach significant concentration in ocular tissues. Drugs in topical drops administered to the corneal surface are mostly washed out by tears into the naso-lacrimal duct. While in the tear film, drugs have limited time to penetrate the cornea to reach the intraocular space. Some drugs may be delivered to the front, anterior portion of the eye by drops but reaching significant therapeutic concentrations in the posterior portion of the eye and the retina is generally not achieved with topical methods of administration.

Many diseases that result in visual loss involve the posterior retina where color vision and reading occur. To treat the posterior portion of the eye and the posterior retina typically drugs are injected into the eye. Sub-conjunctival injections are used to place a drug depot under the outer layer of the eye, however the very high lymphatic flow in the conjunctiva leads to rapid transport of the drug away from the eye. Sub-conjunctival injections are typically not effective to achieving high drug levels in the posterior portion of the eye.

Sub-Tenon's injections are sometimes used to place the drug under the conjunctiva and Tenon's capsule of the eye in a more posterior location to deliver drug to the posterior region of the eye. Sub-Tenon's injections have been demonstrated to be useful for the administration of steroids, however the tip of the injection needle is deep into the posterior shell of the eye where the tip of the needle cannot be directly observed. The technique requires experience and careful technique to avoid physical injury to the eye or misplacement of drug.

Intravitreal injections are given to place drug directly into the vitreous chamber, and typically require a smaller quantity of drug as compared to sub-Tenon's injections. The half-life of the drug is limited due to the fluid in the vitreous which continuously moves forward toward the anterior chamber. This vitreous flow washes out the drug over time and contacts the drug to other tissues of the eye in the flow path. Intravitreally administered drugs such as steroids are associated with complications of cataract progression due to drug exposure to the lens and glaucoma from drug exposure to the trabecular meshwork during flow from the vitreous chamber.

The suprachoroidal space between the choroid and sclera and the supraciliary space between the ciliary body and sclera are more difficult to locate but also can be used for the injection of drugs. Unlike intravitreal injections, the fluid in the suprachoroidal space and supraciliary space flows posteriorly. This flow may assist drugs injected into the suprachoroidal space or the supraciliary space to reach the posterior tissues and posterior retina. Small drug particle sizes are ideal for migration in the suprachoroidal space or supraciliary space however small drug particles release drug at a much faster rate thereby reducing the longevity of the drug treatment.

US 2005/048099 describes biodegradable implants sized for implantation in an ocular region and methods for treating medical conditions of the eye. US2008/131484 discloses biodegradable implants sized and suitable for implantation in an ocular region or site and methods for treating ocular conditions. US2013/289467 discloses drug delivery devices and methods for the treatment of ocular disorders requiring targeted and controlled administration of a drug to an interior portion of the eye for reduction of prevention of symptoms of the disorder.

US2011/238075 discloses methods and devices for use in treating eye conditions with implantable drug delivery devices.

One potential problem with all injections of drug into the eye beneath the sclera is increased intraocular pressure (IOP) caused by the additional volume introduced into the eye. The increased IOP may cause pain and potential damage to the optic nerve. For highly active drugs a small injection volume may be used without significant acute IOP increase, for example 0.05 ml of anti- VEGF drugs. However for larger volumes such as 0.1 ml with steroids, IOP increase may be significant and may cause an acute period of pain and loss of vision.

### Summary of the Invention:

The present invention provides in a first aspect a drug composition for delivery to the suprachoroidal space or supraciliary space comprising a flexible solid elongate body for injection into the suprachoroidal space or supraciliary space, wherein the flexible solid elongate body comprises: (i) a plurality of spherical particles comprising a drug, and (ii) at least one excipient that acts as a binder to form the spherical particles into the flexible solid, wherein said spherical particles comprise a biodegradable or bioerodable polymer that undergoes biodegradation or bioerosion in the suprachoroidal space or supraciliary space after injectionn, wherein said biodegradable or bioerodable polymer is selected from the group consisting of polyhydroxybutyrate, polydioxanone, polyorthoester, polycaprolactone, polycaprolactone copolymers, polycaprolactone-polyethylene glycol copolymers, polylactic acid, polyglycolic acid, polylactic-glycolic acid copolymer and/or polylactic-glycolic acid-ethylene oxide copolymer, and wherein the binder comprises either (i) a water soluble polymer or (ii) a lipid or fatty acid, wherein said binder undergoes dissolution in the physiological conditions of the suprachoroidal space or supraciliary space after injection to allow the spherical particles to disperse and migrate into the suprachoroidal space or supraciliary space, wherein the lipid or fatty acid has a melt transition temperature greater than 20 degrees centigrade and up to 37 degrees centigrade.

The composition may comprise spherical particles comprise 10% to 90% by weight of the drug. The composition may be a flexible solid that comprises approximately 5% to 50% by weight of an excipient that acts as a binder for the drug-containing particles. When the binder comprises a water soluble polymer (i), the water soluble polymer may be selected from the group consisting of polyvinylpyrrolidone, polyvinylpyrrolidone co-vinyl acetate, polyvinyl alcohol, polyethylene glycol, polyethylene oxide or chemically modified cellulose. When the binder comprises a lipid or fatty acid (ii), the lipid or fatty acid may be selected from the group consisting of capric acid, erucic acid, 1,2-dinervonoyl-sn-glycero-3-phosphocholine, 1,2-dimyristoyl-sn-glycero-3-phosphocholine, or 1,2-dipentadecanoyl-sn-glycero-3-phosphocholine.

The drug may be dispersed in the biodegradable or bioerodable material as an amorphous solid dispersion; optionally wherein the drug is dispersed in the biodegradable or bioerodable material as a plurality of drug crystals. The drug particles may additionally comprise a barrier coating.

The flexible elongate body may have a buckling force of less than 670 milligrams of force; optionally wherein the flexible solid may decrease in buckling force or flexural rigidity when in contact with the suprachoroidal space or supraciliary space. The flexible solid may have at least one discrete region of reduced buckling threshold or flexural rigidity along the length of the solid. The discrete region of the flexible solid may comprise a region of reduced cross-sectional area; optionally wherein the discrete region of the flexible solid may comprise a complete cut across the cross-section.

The composition may further comprise a lubricant; optionally wherein the lubricant may be a fatty acid, lipid, sterol or oil.

The drug may be a steroid, non-steroidal anti- inflammatory agent, anti-TNF alpha agent, mTOR inhibitor, cell therapy, neuroprotective agent or nucleic acid based therapeutic wherein (i) the steroid may be dexamethasone, fluocinolone, loteprednol, difluprednate, fluorometholone, prednisolone, medrysone, triamcinolone, betamethasone or rimexolone; (ii) the non-steroidal anti-inflammatory agent may be bromfenac, diclofenac, flurbiprofen, ketorolac tromethamine or nepafenac; (iii) the anti-TNF alpha agent may be infliximab, etanercept, adalimumab, certolizumab or golimumab; (iv) the mTOR inhibitor may be sirolimus, Everolimus, Temsirolimus or a mTOR kinase inhibitor; (v) the cell therapy inhibitor may be mesenchymal cells or cells transfected to produce a therapeutic compound; (vi) the neuroprotective agent may be an antioxidant, calcineurin inhibitor, NOS inhibitor, sigma-1 modulator, AMPA antagonist, calcium channel blocker or histone-deacetylases inhibitor; or (vii) wherein the nucleic acid based therapeutic may be a gene vector, plasmid or siRNA.

The spherical particles may have an average diameter in the range of 5 to 100 microns, and comprise a mixture of diameters to facilitate close packing.

In a second aspect there is provided a drug composition according to the first aspect and an injection device comprising: an elongated barrel with a needle with a lumen at the distal end of the injection device wherein the drug composition of diameter less than or equal to the inner diameter of the needle lumen is contained in the needle lumen; a plunger with a force element that provides an injection force to the drug composition; wherein activation of the force element initiates injection of the drug composition.

In a third aspect there is provided the drug composition of the first aspect for use in the treatment of an ocular disease or condition by injection to the suprachoroidal space or by injection to the supraciliary space. The drug composition may dissolve into a plurality of spherical drug-containing particles; optionally wherein the drug composition may dissolve into a plurality of spherical drug-containing particles that migrate in the suprachoroidal space.

The drug composition may be delivered through a needle or cannula. The drug composition may comprise a steroid, non-steroidal anti- inflammatory agent, antihistamine, aminosterol, antibiotic, VEGF inhibitor, anti-TNF alpha agent, mTOR inhibitor, cell therapy, neuroprotective agent or nucleic acid based therapeutic wherein (i) the steroid may be dexamethasone, fluocinolone, loteprednol, difluprednate, fluorometholone, prednisolone, medrysone, triamcinolone, betamethasone or rimexolone; (ii) the non-steroidal anti-inflammatory agent may be bromfenac, diclofenac, flurbiprofen, ketorolac tromethamine or nepafenac; (iii) the anti-TNF alpha agent may be infliximab, etanercept, adalimumab, certolizumab or golimumab; (iv) the cell therapy inhibitor may be mesenchymal cells or cells transfected to produce a therapeutic compound; (v) the mTOR inhibitor may be sirolimus, Everolimus, Temsirolimus or an mTOR kinase inhibitor; (vi) the neuroprotective agent may be an antioxidant, calcineurin inhibitor, NOS inhibitor, sigma-1 modulator, AMPA antagonist, calcium channel blocker or histone-deacetylases inhibitor; or (vii) the nucleic acid based therapeutic may be a gene vector, plasmid or siRNA.

The ocular disease or condition may comprise inflammation, infection, macular degeneration, retinal degeneration, neovascularization, proliferative vitreoretinopathy, glaucoma or edema.

In keeping with the foregoing discussion, the present disclosure (not according to the invention) provides a drug composition for delivery to the suprachoroidal space, supraciliary space or other tissue spaces of the eye such as the vitreous cavity, subconjunctival space, sub-Tenon's space and sub-retinal space. The drug composition is in the form of a flexible solid or semisolid, shaped as an elongate body for injection. As described herein, the drug composition comprises a plurality of drug-containing particles and at least one excipient to form the drug particles into a flexible solid or a semisolid. As described herein, the excipient comprises a substance that undergoes dissolution in the physiological conditions of the tissue space after injection to allow the drug containing particles to disperse and migrate in the tissue space.

As described herein, the drug-containing particles are in the form of microspheres. The microspheres may have a coating and/or be made with a biodegradable or bioerodable polymer component to modify the rate of drug release. Similarly, the excipient material may be chosen to control the rate of drug release and/or a coating may be applied over the elongated body to modify the rate of drug release.

As described herein, the drug composition comprises a biodegradable or bioerodable material containing a drug and formed as an elongated solid body. The drug may be dispersed in the polymer as an amorphous solid dispersion. The drug may be dispersed in the polymer as a plurality of drug crystals. The drug may be dispersed in the polymer as both an amorphous solid dispersion and as drug crystals.

As described herein, the flexible solid that is formed has a buckling force of less than 670 milligrams of force, which prevents it from inadvertently penetrating the choroid during injection or insertion in the suprachoroidal space or penetrating the ciliary body during injection or insertion into the supraciliary space. The flexible solid may have one or more discrete regions of reduced buckling threshold or flexural rigidity along the length of the solid to promote buckling or lateral deposition of the flexible solid into the injection space. Optionally, the flexible solid may be formulated to decrease in buckling threshold or flexural rigidity when in contact with the tissue space. The flexible solid may have on one discrete region of reduced buckling threshold or flexural rigidity along the length of the solid. Alternatively, the flexible solid may have 2, 3, 4 or 5 regions of reduced buckling thresholds. The regions of reduced buckling thresholds may be distributed substantially evenly along the length of the solid. The discrete region or regions of reduced buckling threshold may take the form of a reduced cross-sectional area or even a complete cut across the cross-section of the solid.

As described herein, the flexible solid or semi-solid shaped as an elongated body is injected into the suprachoroidal or supraciliary space with a device designed for the elongated body. The injection device comprises an elongated barrel with a hollow needle at the distal end, where the lumen of the needle serves as the reservoir for the elongated body and a plunger with a force element such as a spring or gas reservoir that provides an injection force to the elongated body. The elongated body is sized with a diameter less than or equal to the inner diameter of the needle lumen. As described herein, the injection device also comprises a distal element comprising a tissue interface with a distal seal secured to the distal end of the injection device thereby sealing the needle lumen during application of the injection force. The distal seal is penetrable by the distal tip of the needle by the application of pressure on the tissue surface with the distal end of the injection device and the penetrated distal element becomes slidable on the needle to allow advancement of the needle into tissue. Penetration of the distal seal opens a path for delivery of the injection material from the distal end of the needle. The injection device with a force element is activated prior to penetration of the distal seal by the needle and advancement of the needle tip into tissues, thereby enabling simple one-handed operation of the injection device to administer the drug composition shaped as an elongated body to an eye. As described herein, the drug composition shaped as an elongated body is pre-loaded in the injection device, whereby the injection device serves as the storage container for the drug composition prior to use. As described herein, the pre-loaded device is sterilized for use after placement and sealing of the elongated body in the injection device.

These and other aspects of the disclosure will be made apparent from consideration of the following detailed description in conjunction with the accompanying drawing figures.

### Brief Description of the Drawings:

FIG. 1A depicts one description of a plurality of spherical drug containing particles formed into a solid or semisolid and shaped as an elongated body for injection.
FIG. 1B depicts the circled portion of Fig. 1A.
FIG. 2 depicts a comparison of the cumulative drug release profile of drug-containing microspheres and the drug alone.
FIG. 3A depicts one description of a biodegradable or bioerodable material composition formed into a solid or semisolid and shaped as an elongated body for injection containing dispersed drug.
FIG. 3B depicts the circled portion of Fig. 3A.
FIG. 4 depicts the cumulative drug release profile of drug containing microspheres and the drug containing microspheres formed into an elongated solid.
FIG. 5 depicts the cumulative drug release profile of one description of an elongated solid drug composition.
FIG. 6 depicts one description of an injection device for injection of a drug composition formed into a solid or semisolid and shaped as an elongated body for injection into a tissue space.
FIG. 7 depicts the distal end of the injection device in an uncollapsed state.
FIG. 8 depicts the distal end of the injection device in a collapsed state.
FIG. 9 depicts one description of a solid material delivery device.
FIG. 10 depicts one description of a distal tip of a solid material delivery device.
FIG. 11 depicts one description of a distal tip of a delivery device in an uncompressed state.
FIG. 12 depicts one description of a distal tip of a delivery device in a compressed state.
FIG. 13 depicts one description of a distal tip of a delivery device with a collapsible element.
FIG. 14 depicts magnified detail of one description of a distal tip of a delivery device with a collapsible element.
FIG. 15 depicts a plot of the force versus displacement of a distal element with a collapsible element.

### Description of the Invention:

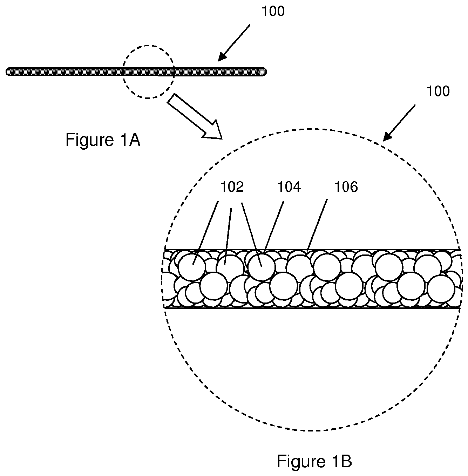

The disclosure (not according to the invention) is a solid or semisolid composition of drug shaped as an elongated body for injection into the suprachoroidal space, supraciliary space or other spaces of the eye such as the vitreous cavity, subconjunctival space, sub-Tenon's space and sub-retinal space. As described herein, the composition comprises a plurality of spherical drug-containing particles 102 formed into a flexible solid or semisolid 100, shown schematically in Fig. 1A and Fig. 1B. For injection of the flexible solid or semisolid in the suprachoroidal space or supraciliary space, the composition is implanted into the eye from the outer surface of the eye through a needle or cannula and the mechanical properties of the flexible solid or semisolid displaces the choroidal tissue under the suprachoroidal space or the ciliary tissue under the supraciliary space to preferentially locate the flexible solid in the suprachoroidal space or supraciliary space near the injection site. After placement in the suprachoroidal space or the supraciliary space, the flexible solid or semisolid transforms or, degrades or dissolves into individual drug containing particles that may migrate in the space. The solid or semisolid mass of drug particles allows a large amount of drug to be injected in a very small volume to prevent an acute increase of intraocular pressure such as occurs with injection of an equivalent amount of drug suspended in a fluid.

The drug may be in the form of microspheres by fabrication of the drug into the form of spherical particles or by the formulation of the drug with a polymer and fabricating microspheres from the combination. Microspheres containing drug may be fabricated by any of the known means for microsphere fabrication such as by spray drying or coacervation. The use of a non-toxic polymer to hold drug within microspheres allows tailoring of the drug release rate by the polymer composition, drug content and size of the microspheres. Microspheres with a drug content of 10-90 weight % drug may provide appropriate drug release. The use of polymers of selected solubility allows both water soluble and water insoluble drugs to be incorporated into microspheres. Suitable polymers include, but are not limited to, non-toxic water soluble polymers such as polyvinylpyrrolidone, polyvinylpyrrolidone co-vinyl acetate, polyvinyl alcohol polyethylene glycol and polyethylene oxide, biodegradable polymers such as polyhydroxybutyrate, polydioxanone, polyorthoester, polycaprolactone, polycaprolactone copolymers, poly lactic acid, poly glycolic acid, poly lactic-glycolic acid copolymers and poly lactic-glycolic acid - ethylene oxide copolymers, and biological polymers such as gelatin, collagen, glycosoaminoglycans, cellulose, chemically modified cellulose, dextran, alginate, chitin and chemically modified chitin.

Alternatively, drug particles of approximately spherical shape or other uniform shapes may be prepared by milling of larger drug particles or controlled crystallization. Drug particles and drug-containing microspheres may also be individually coated with a polymer layer to form drug particles with an external surface coating or barrier coating 104. The coatings may comprise non-toxic water soluble polymers including, but not limited to, polyvinylpyrrolidone, polyvinylpyrrolidone co-vinyl acetate, polyvinyl alcohol, polyethylene glycol and polyethylene oxide, biodegradable polymers such as polyhydroxybutyrate, polydioxanone, polyorthoester, polylactic acid, polyglycolic acid, poly lactic-glycolic acid copolymers, acid terminated polylactic- glycolic acid copolymers, polylactic-glycolic acid-ethylene oxide copolymers, polylactic acid-polyethylene glycol copolymers, polycaprolactone, polycaprolactone copolymers and polycaprolactone-polyethylene glycol copolymers, and biological materials such as gelatin, collagen, glycosoaminoglycans, cellulose, chemically modified cellulose, dextran, alginate, chitin, chemically modified chitin, lipids, fatty acids and sterols.

As described herein (not according to the invention), the plurality of drug-containing particles is formed into a flexible solid with an excipient 106 to act as a binding agent. Suitable binding agents include, but are not limited to, non-toxic water soluble polymers such as polyvinylpyrrolidone, polyvinylpyrrolidone co-vinyl acetate, polyvinyl alcohol, polyethylene glycol and polyethylene oxide, biodegradable polymers such as polyhydroxybutyrate, polydioxanone, polyorthoester, polycaprolactone, polycaprolactone copolymers, polylactic acid, polyglycolic acid, polylactic-glycolic acid copolymers and polylactic-glycolic acid-ethylene oxide copolymers, and biological materials such as gelatin, collagen, glycosoaminoglycans, cellulose, chemically modified cellulose, dextran, alginate, chitin and chemically modified chitin. The drug-containing particles are mixed with the binding agent in a suitable solvent to dissolve or form a dispersion of the binding agent but does not extract the drug from the particles or dissolve the particles. The slurry of drug particles are formed in a mold or extruded and allowed to dry to form a solid 100 of desired dimensions. Ideal for injection of the formed solid is an elongated shape with an outer diameter sized to fit within the lumen of a small gauge needle or cannula, 20 gauge or smaller, corresponding to 0.60 mm diameter or smaller. As described herein, the formed solid has an outer diameter sized to fit within the lumen of a 25 gauge or smaller needle or cannula, corresponding to a 0.26 mm diameter or smaller. As described herein, the drug particles are sized smaller than the inner diameter of the delivery needle or cannula to allow close packing of the drug particles within the formed solid to enhance mechanical properties. Such drug particles would have an average diameter in the range of 5 to 100 microns, for example 10 to 50 microns, and may comprise a mixture of diameters to facilitate close packing. The mean or median diameter of the particles may be in the range of 5 to 100 microns, for example 10 to 50 microns. The mechanical properties of the solid may be tailored by the selection of binder, the amount of binder and by the addition of plasticizers to the binder. To provide the appropriate mechanical properties of the formed solid, the binder may comprise approximately 5% to 50% by weight of the composition (drug composition or drug-polymer combination).

The binder (also referred to as an excipient herein) may be formulated for the appropriate dissolution characteristics to allow dispersion and migration of the drug particles after placement of the formed solid in the tissue space. The binder/excipient can therefore be dissolvable, soluble and/or degradable. In particular, the binder/excipient can be dissolvable, soluble and/or degradable under conditions found at the site of delivery. According to the invention, the binder undergoes dissolution in the physiological conditions of the suprachoroidal or supraciliary space after injection.

The dispersion and migration of the drug particles are desired to promote a uniform distribution of the particles to the eye. The dissolution of the excipient and resultant release of drug particles may be triggered by the absorption of fluid from the tissue space, for example due to the ionic environment or the temperature of the environment. As described herein, the excipient comprises a lipid or fatty acid with a melting temperature between room temperature and the temperature of the ocular tissue space, approximately 37 degrees centigrade (for example, a melting temperature between 21 and 37 degrees centigrade, between 25 and 37 degrees centigrade, or between 30 to 35 degrees centigrade). The rate of release of the individual drug particles from the semisolid may be tailored by the addition of hydrophilic or amphiphilic agents that increase the dissolution rate of the excipients of the solid or semisolid. The release of the drug particles may occur over hours, days or weeks, depending on the amount and composition of the binder. For example, a maximum (or minimum, depending on the formulation) of 50% of the drug may be released after 1 hour, 6 hours, 12 hours, 1 day, 3 days or 1 week.

The solubility of the binder in the fluid of the tissue space may provide dissolution of the binder to release the drug particles from the formed solid. The selection of the binder and its solubility related to the hydrophilicity, molecular weight and crystallinity of the binder may be tailored for the desired dissolution rate. The binding agent may also contain hydrophilic additives such as salts, dextran, or polyethylene glycol to speed water absorption and subsequent dissolution.

Other binding agents may act by the ionic environment of the tissue space to provide dissolution, such as may be provided by ionically crosslinked polymers such as sodium alginate. Other binders may be triggered for dissolution in the tissue space by temperature, such as with lipids and fatty acids with a melt transition temperature greater than room temperature, approximately 20 degrees centigrade, and less than or equal to the temperature within the ocular tissue space, approximately 37 degrees centigrade. Such lipids and fatty acids include, but are not limited to, capric acid, erucic acid, 1,2-dinervonoyl-sn-glycero-3-phosphocholine, 1,2-dimyristoyl-sn-glycero-3-phosphocholine, and 1,2-dipentadecanoyl-sn-glycero-3-phosphocholine and mixtures thereof.

According to the invention, the binder comprises either (i) a water soluble polymer or (ii) a lipid or fatty acid, wherein the lipid or fatty acid has a melt transition temperature greater than 20 degrees centigrade and up to 37 degrees centigrade.

The formed solid 100 comprising the plurality of drug-containing particles 102 may be in the shape of a plug, tube or cylinder. As described herein, the formed solid is an elongated body with a diameter approximately the inside diameter of the needle or cannula used for placement of the formed solid in the tissue space. The diameter may range from 0.60 mm, corresponding to a 20 gauge needle or cannula, to 0.159 mm, corresponding to a 30 gauge needle or cannula. Depending on the dose of drug and drug content of the particles, the formed solid may have a length ranging from 1 mm to 50 mm (for example 1 mm to 25 mm). The volume of the injected formed solid may range from 0.1 microliters to 10 microliters (for example 0.1 to 5 microliters).

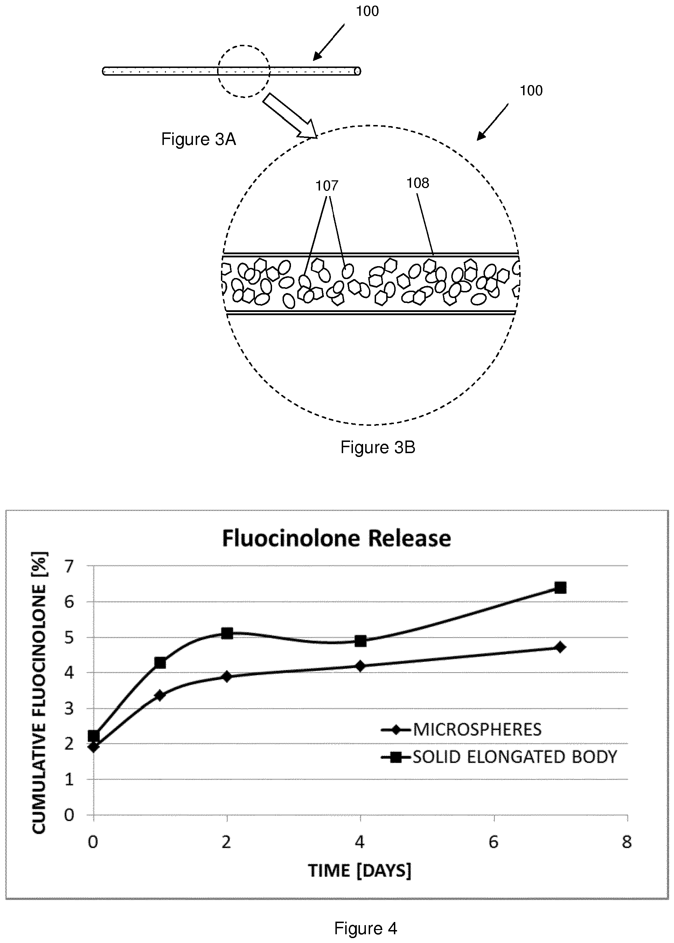

Due to the small size of the drug-containing particles, drug release from the particles may be too rapid to provide sustained drug effect after administration to the eye. It is an object of the disclosure (not according to the invention) to provide drug-containing particles with prolonged release kinetics (i.e. controlled release formulations). As described herein, the drug is incorporated into a polymer matrix that creates a poor diffusion path for the drug thereby slowing drug release as compared to the drug without a polymer matrix. As described herein, the drug particle 102 is coated with a barrier 104 such as a polymer or other compound. The barrier material typically has different chemical properties than the drug so that the drug is not readily soluble through the barrier coating and is slowed in drug release as compared to the drug particle without a barrier coating. One method for selection of the barrier coating is a material with a different partition coefficient or log P than the drug, with an increased difference providing an increased barrier to drug release. As described herein, the individual particles of a drug, are coated with a barrier coating of increased water solubility or decreased log P compared to the drug to form a barrier coating on each particle. Barrier materials may include, but are not limited to, acid terminated poly lactic-glycolic acid copolymers, polylactic acid-polyethylene glycol copolymers and polycaprolactone-polyethylene glycol copolymers. As described herein, the individual particles of a drug are coated with a barrier coating of decreased water solubility or increased log P compared to the drug to form a barrier coating on each particle including, but not limited to, a hydrophobic polymer, lipid, fatty acid or sterol. Drug particles may be coated by any of the known means for particle coating, for example, spray drying, electrostatic spraying or chemical deposition. As described herein, shown schematically in Fig. 3A and Fig. 3B, the formed solid or semisolid material 100 comprises a plurality of drug particles 107 encapsulated or coated with a barrier material 108, such as a soluble polymer or other coating, to modify the drug release characteristics and/or the mechanical properties.

While the drug of the composition is primarily contained in the plurality of particles, some drug may also be formulated into the excipient. The drug in the excipient may act to prevent extraction or diffusion of drug from the particles during processing or storage. The drug in the excipient may also act to provide a rapid release component to the drug formulation to initiate therapeutic effect of the drug while allowing the drug in the particles to provide a sustained delivery to maintain the treatment effect.

As described herein (not according to the invention), the drug composition comprises a drug and an excipient comprising a biodegradable or bioerodable material. The biodegradable or bioerodable material may be comprised of, for example but not limited to, polyhydroxybutyrate, polydioxanone, polyorthoester, polycaprolactone, polycaprolactone copolymer, polycaprolactone-polyethylene glycol copolymer, polylactic acid, polyglycolic acid, polylactic-glycolic acid copolymer, acid terminated polylactic-glycolic acid copolymer, or polylactic-glycolic acid-ethylene oxide copolymer, gelatin, collagen, glycosoaminoglycan, cellulose, chemically modified cellulose, dextran, alginate, chitin, chemically modified chitin, lipid, fatty acid or sterol. The drug may be dispersed in the biodegradable or bioerodable material as an amorphous solid dispersion. The drug may be dispersed in the biodegradable or bioerodable material as a plurality of drug crystals. The drug may be dispersed in the biodegradable or bioerodable material as both an amorphous solid dispersion and as drug crystals. The drug composition is shaped as an elongate solid body for injection into the ocular tissue space. Release of the drug from the elongated body allows the drug to diffuse into the tissues of the eye and may be assisted by the flow of fluid in the tissue space. In the case where the drug is in the form of a solid amorphous dispersion, the biodegradable or bioerodable material is selected to provide the desired drug loading and release characteristics of the drug. In the case where the drug is in the form of dispersed drug crystals, the amount of drug, the biodegradable or bioerodable material characteristics and the crystal form of the drug may be selected to provide the desired drug loading and release characteristics of the drug. The drug crystals may also be coated with an excipient to reduce the drug release rate of the drug composition.

To place the composition into a tissue space, the composition shaped as an elongate body is placed in an injector device comprising a needle or cannula of appropriate inner diameter where the composition is a material for injection by the injection device.

The device comprises an elongated body with a hollow needle at the distal end, a slidable plunger at the proximal end, and a reservoir for the material to be injected residing between the needle and the plunger. The reservoir is configured to receive an injection material to be delivered through the needle. In particular, the injection material is the drug composition of the present disclosure.

The plunger acts to push the injection material through the needle to the desired tissue location. A plunger with a force element is configured to provide an injection force to the injection material. The force element may comprise a spring mechanically coupled to a plunger. The force element may be at least partially within the elongated body of the device. The plunger is mechanically coupled to a source of force such as a spring or pressurized gas reservoir such that an injection force is applied to the injection material within the device after the injection material is placed in the reservoir and prior to insertion into ocular tissues and prior to injection of the injection material into an eye. Secured to the distal end of the needle is a distal element comprising a distal seal, which also acts as a tissue interface. The distal element is moveably secured to the distal tip of the needle where it serves to close off the distal end of the needle to close the path of the injection material from the needle tip. As described herein the distal element has a lumen to fit over the outer diameter of the needle. As described herein the distal element is secured to the distal tip of the needle through other means. The distal seal of the distal element is distal to the tip of the needle and is configured to be penetrated by the needle as the device is placed on the surface of the eye and is compressed by the user. The needle penetrates the distal seal and inserted into ocular tissue, thereby opening a flow path or path of delivery of the injection material from the reservoir, through the needle and into the eye. The resulting self-actuating injection mechanism insures opening of the delivery path for the injection material immediately when the needle is placed in tissue, regardless of the orientation and speed of needle insertion.

As described herein, the distal element comprises a tissue interface and distal seal mounted on a tubular distal housing. The tubular distal housing is fit to the exterior of the needle and may be sealed to the surface of the needle at some point along its length. As described herein, the housing may be sealed by means of an elastomeric element which is compressed between the housing and the needle. The elastomeric element may therefore be annular. As described herein, the elastomeric element may be compressed between the housing and the body of the device. The elastomeric element may reside at or near the proximal end of the housing. As described herein, the elastomeric element serves as a seal between the housing and the needle. As described herein, the elastomeric element serves as a frictional element or component which limits the housing travel in the proximal direction to thereby apply a force against the tissue surface by the tissue interface as the needle penetrates the tissues. As described herein, the distal element comprises a tissue interface and a distal seal and is slidably attached to the exterior of the needle without a distal housing.

The distal element, which comprises a tissue interface with a distal seal, or a tissue interface with a distal seal and an attached housing, is attached to the distal tip of the needle but is not freely movable or slidable proximally from the end of the needle due to the closed distal seal. After the injection material is loaded into the device, the material comes under pressure from the source of force but cannot move through the distal seal. The tissue interface is placed on the surface of the eye and the device is manually advanced, thereby forcing the needle through the distal seal and then through the external surface of the eye into underlying tissues. The distal element after penetration of the distal seal becomes proximally slidable from the end of the needle to retain the tissue interface on the surface of the eye during advancement of the needle into tissue. When the distal tip of the needle penetrates through the distal seal, the source of force immediately allows for expression of the injection material from the needle tip and into the tissues.

As described herein, the tissue interface and distal seal is secured to a housing disposed about the needle. The housing may be comprised of a cylindrical element which is secured to the distal end of the body of the device at the proximal end of the housing. The housing may contain collapsible, distortable or deformable elements which allow the distal end of the housing to retract slidably along the needle, which in turn allows the needle tip to penetrate the distal seal. As described herein the distal element is secured to the distal tip of the needle through other means.

As described herein, the device comprises an elongated barrel with a hollow needle at the distal end and a slidable plunger at the proximal end. The lumen of the needle serves as the reservoir for the injection material shaped as an elongated body with a diameter less than or equal to the inner lumen of the needle. The plunger can be actuated either manually or with a force element such as a spring or pressurized gas source, to push the plunger and eject the injection material into the tissue space when the distal tip of the device reaches the space.

Operation of the device mechanism opens the path for the injection material to move out from the tip of the needle immediately upon penetration through the distal seal which occurs just prior to the entry of the needle into the target tissue. Since the injection material is under pressure prior to penetration of the distal seal by the needle tip, the injection is triggered solely by placement and subsequent advancement of the needle through the tissue interface. This allows precise and automatic control of the timing of the injection action solely due to the needle tip entering the target tissue. The resultant self-actuated mechanism obviates the need for a separate control mechanism, for example a valve or trigger on the body of the injection device, and hence allows for administration of the injection material without the need for special positioning of the fingers or the use of the second hand. The device thereby enables an injection to be performed with a single hand, allowing the other hand of the physician to stabilize the eye or perform other actions to facilitate injection. The self-actuating injection mechanism also eliminates the need for the user to determine when to begin injection which is especially useful when the target tissue space is difficult to locate due to small target size, lack of visualization and anatomic variability.

The device allows precise control of the position of the needle by the user during use. The needle is fixed to the body to the device to allow direct control of the distal tip of the needle when the device is held by the body. Since the injection force is provided by the force element, the plunger of the device does not have to be held, triggered or actuated by the hand holding the device, allowing the device to be held and used in a natural, highly controllable position such as with a writing instrument or scalpel. Generally, the needle is arranged parallel to the elongated body or barrel of the device.

Once the device is activated by penetration of the distal seal and insertion into the eye, the injection material cannot flow or move into the eye until a space to accept the injection material is reached by the distal end of the needle. Scleral tissue in particular is very resilient and effectively seals the needle tip during passage of the needle tip to the suprachoroidal or supraciliary space, hence the unique properties of the sclera do not allow for the injection material to enter the sclera. Once an underlying space such as the suprachoroidal space or the supraciliary space is reached by the needle, the injection material is able to flow or move out of the needle to be delivered. By this mechanism the injection material is directed to a location that can accept the injection material at the distal tip of the needle. The delivery of the injection material may be further directed by the tissue interface. The tissue interface may optionally apply a force to the surface of the eye to aid sealing of the needle tract at the surface of the eye. With an appropriate needle length and orientation, the device may be used to inject into the sub-conjunctival space, sub-Tenon's space, suprachoroidal space, supraciliary space, sub-retinal space, the vitreous cavity, or the anterior chamber.

The injection material is general a solid or semi-solid material, in particular the flexible drug composition of the disclosure. The material may be loaded into the lumen of the needle with the distal end of the solid or semi-solid in contact with the distal seal provided by the distal element at the distal end of the needle. The needle may extend proximally in the body of the device to provide an extended length of the injection material. A plunger is inserted into the proximal end of the needle and the distal end of the plunger is put into contact with the proximal end of the injection material. Placement of the plunger preloads a force element such as a compression spring acting on the plunger to provide an injection force on the material. As described herein, the force element is self-contained in the device or is integrated on the body of the device. The injection material can be placed into the reservoir portion of the device, in a manner similar to a syringe, through a connector, valve or septum in fluid connection to the reservoir. Placement of the injection material in the device preloads a force element such as a compression spring acting on the plunger providing an injection force on the injection material in the reservoir. Other mechanisms may be provided for activating the injection force. For example, the injection force may be activated by a mechanism to compress the force element from the exterior of the device. In another option, the injection force may be activated by mechanically releasing a constrained force element or gas prior to use.

The size of the reservoir, needle and plunger may be sized appropriately for the volume of injection material to be delivered. For the solid or semi-solid injection material of the disclosure, the needle and potentially an extension of the needle into the body of the device may act as the reservoir. The needle and plunger may be sized for solid or semi-solid delivery volumes ranging from, for example, 0.1 microliters to 8 microliters.

The needle comprises a stiff material with a diameter to allow the injection material to pass through the lumen of the needle, typically in the range of 20 gauge to 40 gauge (for example less than 0.91 mm outer diameter / 0.6 mm inner diameter), where the length of the needle is suitable to both reach the intended tissue and provide sufficient volume for the desired dose of the composition to be administered. The needle is fixed to the body or barrel of the device and generally does not slide or move in relation to the body to provide precise control of needle depth during penetration of tissues. The needle may extend proximally into the body of the injection device to provide sufficient volume for the injection material.

The distal tip of the needle may be beveled or sharpened to aid penetration. The bevel angle may be designed to facilitate entry into a specific target. For example, a short bevel of 18 degree bevel angle may be used to inject into narrower spaces such as the subconjunctival or sub-Tenon's space. A medium bevel needle of 15 degree bevel angle may be used to inject into spaces such as the suprachoroidal or supraciliary space. Longer bevels, such as 12 degree bevel angle may be used to inject into the anterior or posterior chambers.

As described herein, the distal element is designed with a complementary bevel in a lumen of the distal element to provide close apposition of the distal seal to the needle bevel. The bevel of the needle is in alignment with the bevel in a lumen of the distal element. The most distal portion of the distal element may be flat or beveled to aid orientation of the needle during tissue penetration to aid reaching certain injection targets. For example, a beveled tissue contacting surface of the distal element may aid targeting of injections into the tissue targets with less injection depth such as the subconjunctival space, sub-Tenon's space and in some regions of the suprachoroidal space. The angle of the tissue contacting surface of the distal element may range from 90 degrees from the axis of the distal element for perpendicular insertion, to 15 degrees from the axis.

The needle may be constructed from a metal, ceramic, high modulus polymer or glass. The length of the needle in tissue is selected to match the target location for the injection and the variation in target location due to anatomical variability. The effective full length of the needle is the length of the needle distal tip to the distal surface of the tissue interface, when the distal element has achieved full proximal travel. The distal element moves slidably on the needle during injection, allowing for progressive increase in the length of needle protruding through the distal element during advancement into tissue. The injection material is injected automatically once the needle reaches the appropriate location which may be less than the effective full length of the needle. The release of force and resultant time for injection occurs quickly, in approximately 0.1 to 2 seconds depending on the properties of the injection material and the amount of force from the plunger force element. The time for injection may also be controlled by a damping or frictional mechanism coupled to advancement of the plunger to limit the speed of the plunger. The release of force from the force element communicates to the physician with both visible and tactile feedback that there is no need for additional advancement of the needle. The rapid injection event gives the physician sufficient time to halt needle advancement, resulting in an effective variable needle length to accommodate patient to patient differences in tissue thickness. The variable needle length and self-actuation of injection is especially useful for injection into spaces that are not normally open spaces, such as the subconjunctival space, sub-Tenon's space, suprachoroidal space and supraciliary space. For the subconjuctival space and sub-Tenon's space the needle effective full length is in the range of 0.35 mm to 2 mm depending on the angle of needle insertion. For the suprachoroidal space and supraciliary space, the needle effective full length is in the range of 1 mm to 4 mm depending on the angle of insertion. For the vitreous cavity, the needle effective full length is in the range of 10 to 15 mm. The effective full needle length may, for example, be 0.3 mm to 3 mm, 0.35 to 2 mm, 1 mm to 4 mm, 10 to 15 mm.

As described herein, the distal element applies a distally directed sealing force against the tissue surface to maintain a seal on the surface of the eye. As described herein, the distal element maintains contact with the tissue surface but does not apply a distally directed sealing force against the tissue surface to maintain a seal on the surface of the eye. As described herein, the distal element contacts the surface of the eye during penetration of the distal seal of the distal element by the distal tip of the needle but does not maintain contact with the surface of the eye after needle penetration through the distal seal and into ocular tissue. The tissue interface and distal seal may comprise a soft polymer, rubber or other material that allows needle penetration without coring of the material. The tissue interface and distal seal material is selected to provide compliance to both seal to the surface of the eye during insertion of the needle into ocular tissue and also to seal the injection pathway from the needle until the needle is advanced through the distal seal. Once the needle penetrates the distal seal, the needle is advanced through the outer ocular tissues to reach the desired injection site. The tissue interface and distal seal remain on the surface of the eye. The distal seal is sufficiently resilient to prevent rupture by the injection material under pressure prior to advancement of the needle through the distal seal. The portion of the distal seal in the path of the needle is also sufficiently thin to allow penetration by the needle without undue application of force. The distal seal is typically in the range of 250 to 1500 microns in thickness in the region that is penetrated by the needle.

As described herein, a sealing force is provided by a compression spring between the body of the device and the proximal end of the distal element or distal housing. As described herein, the tissue interface provides a sealing force by compression of the tissue interface or elastically compressible elements in the distal element. As described herein, the distal element is configured to allow an elastic reduction in length during needle advancement to apply a sealing force. As described herein, a friction element disposed in or about the distal element increases the force required to move the distal element proximally thereby promoting contact of the tissue interface with the surface of the eye and maintaining a seal against the eye surface during needle advancement. The friction of the distal element against the needle may be tailored in relation to the proximal movement of the distal element during needle advancement. An increase in friction may be obtained by increased contact or surface texture between the distal element and the external surface of the needle to tailor the amount of force applied by the tissue interface during proximal travel of the interface along the needle length. The friction may be varied along the path of travel of the distal element along the needle. High friction may be provided during the initial path of travel of the distal element to promote contact of the tissue interface to the surface of the eye during initial insertion of the needle into ocular tissues, the friction may be reduced after a length of the needle corresponding to the length of the needle bevel is inserted into ocular tissue. The length of travel of the distal element under the influence of the region of high friction is in the range of 0.3 mm to 2 mm.

As described herein, the distal element is attached to the body of the device by one or more collapsible elements. The collapsible element is configured to not allow an increase in length to prevent the distal seal from being displaced from the tip of the needle due to the injection force applied to the injection material prior to penetration of the distal seal. The collapsible element allows a reduction in length, thereby allowing proximal travel of the distal element during advancement of the needle into tissues. As described herein, the collapsible element comprises one or more elongated struts that may deform, bend or fold away from the needle during proximal travel of the distal element. As described herein, the collapsible element comprises a section of tubing concentric to the needle that has been cut to form openings along the axial length of the tubing to form collapsible struts. The shape and configuration of the collapsible struts may be tailored to provide a desired force-displacement characteristic of the collapsible element. As described herein, the collapsible element provides a sealing force which transitions from an increasing spring like force per unit displacement to a constant force independent of displacement to keep the tissue interface and distal seal in sealing contact to the eye surface without undue application of force with further needle advancement into the eye. The transition to a constant force is designed to occur after a length of the needle bevel is inserted into ocular tissue, corresponding to a compression or collapse of the collapsible element of 0.3 mm to 2 mm. As described herein, the collapsible element provides for contact of the tissue interface to the surface of the eye during initial insertion of the needle into ocular tissue but collapses to provide little or no resistance to proximal movement of the distal element along the needle after the bevel of the needle is fully inserted into tissue. The collapsible element may be assembled from components in a tubelike configuration or alternatively cut from a segment of tubing such as a laser machined nickel titanium alloy (nitinol) tube. The collapsible element may be disposed between the elongate body and the distal element, such as between the barrel and the housing of the distal element (if present).

Suitable materials for the tissue interface and distal seal include, but are not limited to, natural rubbers, silicone rubbers and thermoplastic elastomers such as polyurethanes. The stiffness of the rubber or elastomer may be selected to provide the appropriate combination of conformance to the tissue surface and sealing of the lumen of the distal end of the needle. The rubber or elastomer must also be capable of penetration by the distal tip of the needle to trigger release of the injection material. Rubbers or elastomers with a Shore A durometer of 25 to 90 are suitable for use as the sealing element. Suitable materials for a distal housing include, but are not limited to, polypropylene, polyethylene, polycarbonate, polysulfone, polyetheretherketone, acrylonitrile butadiene styrene, polystyrene, polyamide, and polyurethanes. Suitable materials for a distal collapsible element include, but are not limited to, stainless steel, spring temper steel, super-elastic nickel titanium alloys, cobalt chrome alloys, oil-tempered chrome silicone, and polyetherimide. As described herein, the barrel of the device contains the reservoir and provides an external surface for holding the device during use. The reservoir may comprise a tubular cylinder attached on the distal end to the proximal end of the needle, with a plunger slidably disposed in the lumen of the tubular body. The reservoir may also provide for insertion of a cartridge containing the injection material where the plunger of the device moves a slidable seal in the proximal end of the cartridge to deliver the injection material. The body may be fabricated from a variety of thermoplastic materials suitable for medical use such as polypropylene, polycarbonate, polysulfone, polyethylene, cyclic polyolefins, polystyrene and polymethylmethacryate. The body may incorporate external features such as textures or finger indentations to allow a user to more ergonomically grip and use the device. The body may incorporate index or measurement markings to provide an indication of the amount of material being delivered. The body many incorporate transparent materials or a section of transparent material to allow the visualization of the injection material in the reservoir or movement of the plunger to visually indicate the injection event. The plunger may have markings to aid visualization of reservoir loading and release of injection material.

In a description of the disclosure, the device comprises a means for providing an injection force. Said means as described herein could be, for example, a syringe with a compressible reservoir that can be "squeezed" or compressed by a user (directly or indirectly) to effect injection of material. Alternatively, in a preferred description, the means is a plunger with a biasing means or force element (such as a compression spring or a pressurised gas).

The device may be disposable and/or for single use. Alternatively, the device may be reusable.

The distal seal acts to prevent escape of the injection material from the needle or reservoir when the device is primed (by insertion of injection material into the reservoir) prior to activation by a user. This can be achieved by a hermetic seal between the needle lumen and the outside of the device. This hermetic seal may be achieved by the seal being in direct contact with the needle tip or may be achieved by using a distal element housing that is suitably sized to provide a liquid-tight seal around the needle shaft when placed over the needle tip. For example, the outer diameter of the needle may be complimentary to the inner diameter of the housing to provide a seal.

The person skilled in the art will appreciate the difference between flowable, semi-solid and solid injection materials. Any injection material may be described as flowable if, for example, the kinematic viscosity of the material is less than about 0.002 m² / s at 20 °C. An injection material may be described as semi-solid if, for example, the kinematic viscosity of the material is greater than about 0.002 m² / s at 20 °C.

Generally speaking, and as described above, the device is primed since a pressure or force is placed on the injection material such that once the distal seal is penetrated by the needle and the needle reaches the desired site of delivery in the eye (such as the suprachoroidal space or supraciliary space), the injection material is automatically released. In this way, the device can be operated with one hand. The only force that needs to be applied by the user is the penetration force to allow the needle to penetrate the distal seal and then the eye tissue. The needle length can be suitably designed to target specific injection sites at corresponding depths in the eye. As described herein, the device may comprise a retaining means to retain the distal element on the needle once the device is primed.

Prior to injection of the material, the distal element will generally not be in direct physical contact with the elongate body or the barrel. In fact, the distance between the proximal end of the distal element and distal end of the elongate body or barrel (and design of any compressible element that may be present) can be arranged to determine the maximum depth of injection. For example, during operation of the device, as the distal seal is pressed against the eye, the distal element and elongate body or barrel will move towards each other. It is this motion that advances the needle tip towards and through the distal seal/tissue interface and into the patient's eye. Once the proximal end of the distal element abuts against the distal end of the elongate body or barrel (or once the compressible element does not permit further compression), continued advancement of the needle is prevented. Hence, the distance between the proximal end of the distal element and distal end of the elongate body or barrel may be equal to the maximum depth of injection. Account may need to be taken for any distance between the needle tip and the distal seal/tissue interface and/or the use of any compressible element. In particular, the maximum depth of injection may be determined by the distance between the proximal end of the distal element and distal end of the elongate body or barrel less the distance between the needle tip and the distal seal/tissue interface. Thus, the position and sizes of the distal element, needle, and distance between the needle tip and distal seal/tissue interface (if any) can be configured to determine a maximum injection depth. The skilled person could design the device accordingly based on the present disclosure.

In this way the device may comprise a means for determining a maximum injection depth to control the depth of injection of the needle (and hence injection material) into the eye. Said means can be a set distance between the proximal end of the distal element and distal end of the elongate body or barrel (as determined by the relative size of the distal element, the needle, the distance of the needle tip from the distal seal/tissue interface, and the shape and configuration of any compressible element present). Alternatively, the needle may comprise a separate element that halts advancement of the distal element along the needle during operation (such as an element present on the needle disposed between the distal element and the elongate body or barrel, for example an annular ridge or clamp). As described herein, this element to prevent further advancement of the distal element along the needle during operation may be moveable such that injection depth can be determined by the user. As described herein, the needle may comprise markings to allow the use to select an appropriate injection depth. As described herein, the depth of injection may be determined by the compressible element, for example said compressible element only allowing the desired injection depth by way of increasing rigidity as the element is compressed, or by other mechanical means, such as entrapment of the compressible element between the proximal end of the distal element and distal end of the elongate body or barrel. The present disclosure therefore provides devices having fixed maximum injection depths suitable for targeting the tissue of interest. Suitable designs to achieve a fixed maximum injection depth would be apparent to the skilled person based on this disclosure. Of course, the depth of injection can be within certain tolerances. Injection depth is also referred to herein as effective needle length.

As described herein, the drug containing composition shaped as an elongated body is pre-loaded in the injection device, whereby the injection device serves as the storage container for the drug composition prior to use. As described herein, the pre-loaded device is sterilized for use after placement and sealing of the drug containing composition in the injection device. The sterilization may be accomplished by established methods of sterilization such as heat or ionizing radiation.

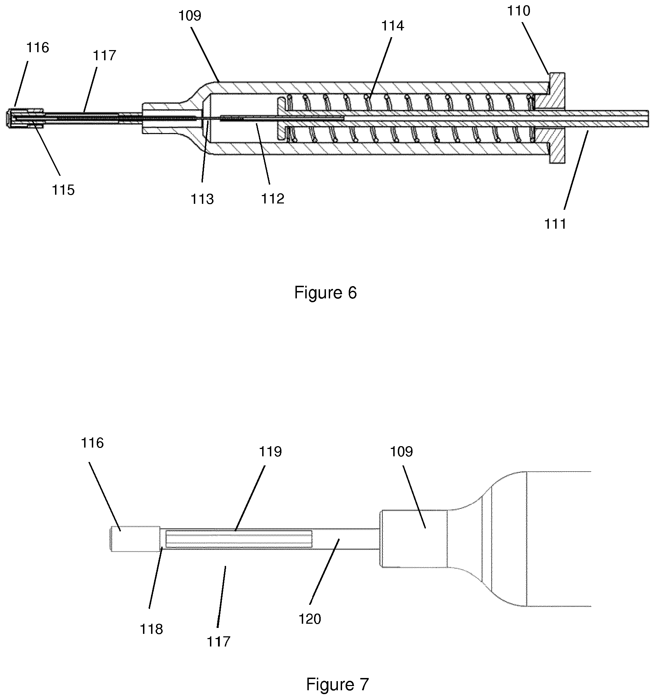

One description of the injection device configured to deliver an elongated solid or semi-solid material within the lumen of a needle is depicted in Fig. 6. The device comprises a hollow barrel 109, with a proximal barrel end cap 110. A plunger 111 slidably passes through the end cap. A push shaft guide tube 112 is slidably disposed in a lumen in a plunger 111, which provides support for a push shaft 113 to prevent the push shaft from buckling during injection. A plunger compression spring 114, serves as the force element and provides a distally directed force on the plunger 111 and push shaft 113. A beveled needle 115, is attached and fixed to the distal end of the barrel 109, such that the needle 115 does not move in relation to the barrel 109 to provide direct control of the location of the needle 115 tip when manipulating the position of the barrel 109. The elongated solid body is retained inside the lumen of the needle 115. The distal end of the push shaft 113 resides within the lumen of the needle 115 and moves distally when the tissue interface and distal seal 116 is opened by the distal tip of the needle 115. The distal element comprises a tubular distal housing 117 surrounding the distal end of the needle 115. The tissue interface and distal seal 116 is attached to the distal end of the distal housing 117.

The distal housing 117 is comprised of distal segment, a central collapsible segment and a proximal segment. Figure 7 shows the distal end of the device in the uncollapsed state. The tissue interface and distal seal 116 is disposed about a distal tubular shaft 118. The central segment is comprised one or more segments 119 which function as collapsible elements which can optionally impart a force against the tissue surface during use. The collapsible elements 119 are attached or integral to the distal tubular shaft 118 and proximal tubular shaft 120.

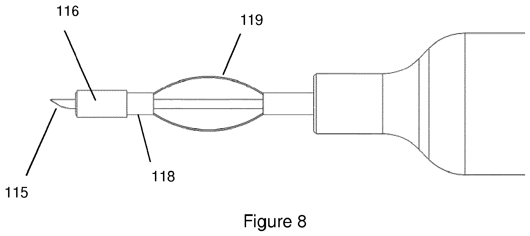

The proximal tubular shaft 120 is connected to the barrel 109 of the device providing an anchor point for the collapsible element and preventing distal movement of the tissue interface and distal seal 116. Fig. 8 shows the distal end of the device in a collapsed state. The force of advancing the device into the tissue causes the collapsible elements 119 to deform, allowing the distal tubular shaft 118 and tissue interface and distal seal 116 to slide proximally along the needle 115. The distal tip of the needle 115 has penetrated the tissue interface and distal seal 116.

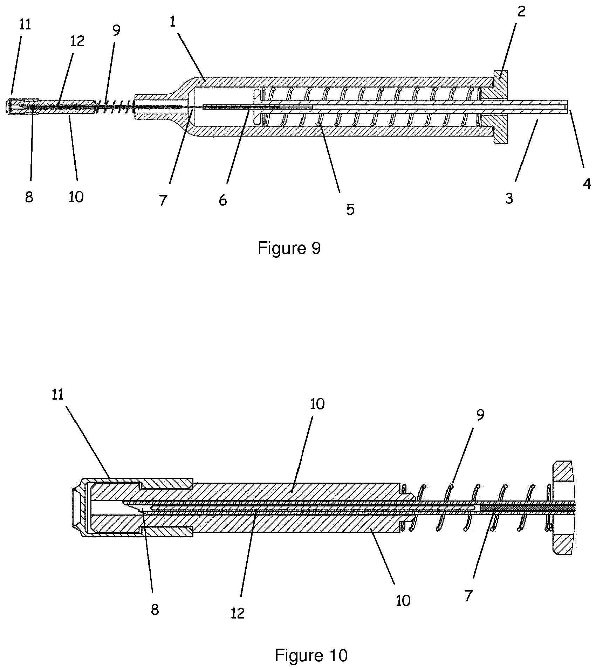

As described herein, the device is configured to deliver an elongated solid or semi-solid material or implant within the lumen of the needle. Referring to the device depicted in Fig. 9 and the distal tip detail of the device in Fig. 10, the device comprises a hollow barrel 1, with a proximal barrel end cap 2. A plunger 3 slidably passes through the end cap. The plunger has a proximal end 4 which is sealed. A push shaft guide tube 6 is slidably disposed in a lumen in the plunger 3, which provides support for a push shaft 7 to prevent the push shaft from buckling during injection. A plunger compression spring 5, provides a distally directed force on the plunger 3 and push shaft 7. A beveled needle 8, is attached and fixed to the distal end of the barrel 1, such that the needle 8 does not move in relation to the barrel 1 to provide direct control of the location of the needle 8 tip when manipulating the position of the barrel 1. The distal end of the push shaft 7 resides within the lumen of the needle 8 and moves distally when the tissue interface and distal seal 11 is opened by the distal tip of the needle 8. The distal element for the needle 8 comprises a tubular distal housing 10 surrounding the distal end of the needle 8. The tissue interface and distal seal 11 is attached to the distal end of the distal housing 10. A distal housing spring 9, is placed between the distal end of the barrel and the proximal end of the distal housing 10 to provide a distally directed force on the distal housing thereby pressing the tissue interface and distal seal 11 onto the tissue surface.

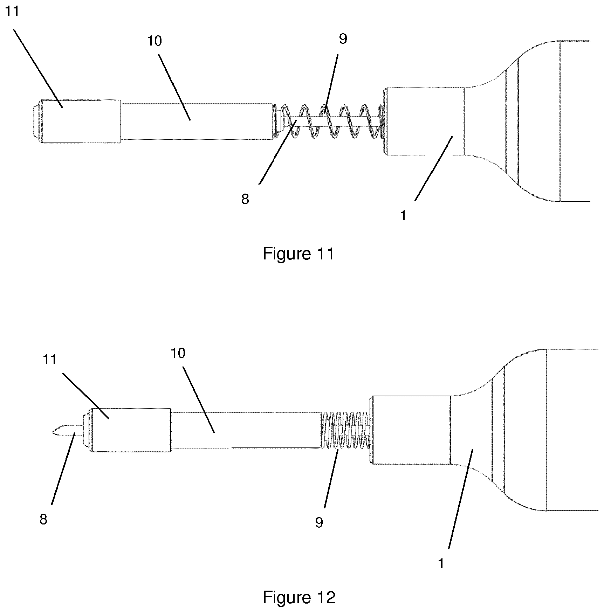

As the needle 8 is advanced to penetrate the tissue interface and distal seal 11, the distal housing 10 moves proximally toward the barrel 1 while the needle 8 is advanced into tissue. The distal housing spring 9 acts to maintain pressure of the tissue interface and distal seal 11 as the needle 8 is advanced into tissue. While the tip of the needle 8 is passing through the outer tissues of the eye, the solid or semi-solid injection material or implant 12 within the lumen of the needle 8 is under pressure from compression spring 5 and the path from the distal tip of the needle 8 has been opened, but there is no tissue space for the injection material 12 to be delivered from the needle tip. Once the distal tip of the needle reaches the desired space such as the suprachoroidal space, the supraciliary space or the vitreous cavity the injection material 12 can exit from the needle and is expelled into the space. Fig. 11 shows the distal segment of the device in an uncompressed state. The tissue interface and distal seal 11 and the distal housing 10 are disposed at the end of the uncompressed distal spring 9. The distal spring 9 is anchored to the barrel 1. Fig. 12 shows the distal segment of the device in a compressed state. The force of advancing the device into the tissue causes the distal spring 9 to compress, allowing the distal housing 11 and distal seal and interface 11 to slide proximally along the needle 8. The distal tip of the needle 8 has penetrated the tissue interface and distal seal 11.

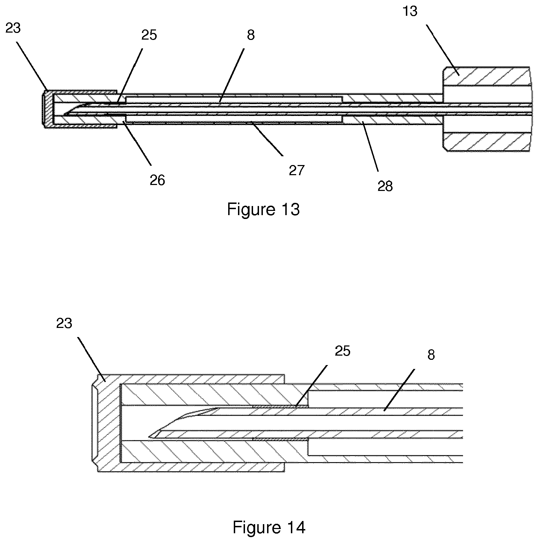

As described herein, the distal tip of the device is comprised of collapsible elements. Referring to the device depicted in Fig. 13 and the magnified device distal tip detail in Fig. 14, the distal tip is comprised of distal segment, a central collapsible segment and a proximal segment. The tissue interface and distal seal 23 is disposed about a distal tubular shaft 26. The inner lumen of the distal tubular shaft 26 contains an internal seal 25 which seals the space between the tubular distal shaft 25 and the bevelled needle 8. The central segment is comprised one or more segments 27 which function as collapsible elements which can impart a force against the tissue surface during use. The collapsible elements 27 are attached or integral to the distal tubular shaft 26 and proximal tubular shaft 28. The proximal tubular shaft 28 is connected to the barrel 13 of the device providing an anchor point for the collapsible element and preventing distal movement of the tissue interface and distal seal 23. Figs 7 and 8, described above, provide further details of the collapsible element.

The descriptions of the device may be used in combination to deliver a solid, semi-solid or liquid. The configuration of the distal portion of the device comprises the distal element comprising the tissue interface and distal seal on the distal end of the needle. The use of a distal compression spring, frictional element, a collapsible element, or a combination of such elements in conjunction with the distal element may be used for delivery of a solid, semi-solid or liquid.

For use in the device for delivery of a solid or semi-solid, a lubricant may be used to aid injection. The lubricant may be used to coat the solid or semi-solid injection material or the needle lumen. The lubricant may also be placed in the lumen of the distal element to coat the tip of the injection material and the outer surface of the needle as it passes into tissue. Suitable lubricants include, but are not limited to, oils, waxes, lipids, fatty acids and low molecular weight polymers. Low molecular weight polymers include, but are not limited to, polyethylene glycol and polysiloxane.

A variety of drugs may be delivered by the present disclosure to the eye for use in the treatment of ocular diseases and conditions including inflammation, infection, macular degeneration, retinal degeneration, neovascularization, proliferative vitreoretinopathy, glaucoma and edema. Useful drugs include, but are not limited to, steroids, non-steroidal anti-inflammatory agents, antibiotics, VEGF inhibitors, PDGF inhibitors, anti-TNF alpha agents, mTOR inhibitors, cell therapies, neuroprotective agents, anti-hypertensive agents, antihistamines, aminosterols and nucleic acid based therapeutics. The drugs may be in the form of soluble solutions, suspensions, gels, semi-solids, microspheres or implants. As described herein, the drug is preloaded in the device prior to use during the time of manufacture. The source of force to provide an injection force to the injection material may be activated just prior to use. As described herein, the activation is achieved by a mechanism to preload the force element, such as compressing a spring, from the exterior of the device such as by a movable proximal handle attached to the plunger. As described herein, the source of force is preloaded during manufacture when the drug is placed in the device and the preloaded force is stabilized by means of a stop mechanism. Prior to use, the stop mechanism is released, thereby placing the force on the injection material prior to contact or penetration of the eye and the injection is triggered by the advancement of the needle through the tissue interface and distal seal as with the previous descriptions of the disclosure.

As described herein (not according to the invention) there is provided an injection device for solid or semi-solid injection material (in particular the drug composition of the disclosure) comprising an elongate body having a hollow needle at its distal end. The distal end of the needle is housed in a distal element having a distal seal that seals the lumen on the needle preventing any injection material from being released. The device also comprises a reservoir formed from the lumen of the needle. The device is primed by introduction of injection material into the reservoir. The device has a collapsible element housed between the distal element and the elongate body, which serves to retain the distal element on the needle, whilst allowing compression of the distal element along the length of the needle and toward the elongate body when the device is activated. In use, the distal element is placed on the surface of the eye and a pressure applied by the user. This causes the needle tip to advance towards and penetrate the distal seal, thereby allowing injection material to be dispensed from the distal end of the needle. However, the material will only be dispensed once the needle tip reaches a void in the target tissue of the eye. The pressure applied on the injection material when the device is primed allows injection of the material to the target site automatically and with the use of only one hand. The needle length and/or length of the collapsible element (and hence distance between the proximal end of the distal element and elongate body) can be configured appropriately to target different spaces at different depths within the eye.

The mechanical properties of the composition are important for the successful placement of the composition. For injection into the suprachoroidal space or supraciliary space, the material must have compressional properties such that it does not penetrate the choroid underlying the suprachoroidal space or the ciliary body underlying the supraciliary space and create hemorrhage, or continue to penetrate into the vitreous cavity or anterior chamber. As described herein, the drug composition is a flexible formed elongated solid, where the solid is designed to deflect at the surface of the choroid or ciliary body. The distal end of the formed elongated solid is tailored to buckle when advanced in contact with the choroid or ciliary body. Continued advancement of the formed elongated solid results in bending, folding or lateral displacement along the length of the formed elongated solid to retain the formed elongated solid in the suprachoroidal space or supraciliary space. Testing has demonstrated a buckling force of a 2 cm length elongated solid of less than 670 mg of force to be suitable for the formed elongated solid. A formed solid in the range of 24 mg of force to 634 mg of buckling force was shown to be suitable to prevent penetration of the choroid. As described herein, the formed elongated solid rapidly hydrates in contact with the fluids of the tissue space to result in a decrease in mechanical properties such as buckling force or flexural rigidity so that the formed elongated solid folds or coils in the tissue space. As described herein, the formed elongated solid has discrete regions of lower flexural rigidity along the length such as reduction in diameter, a partial or complete cut across the cross section, or an area of lower concentration of drug-containing particles. The discrete regions provide means for the formed elongated solid to transition into a series of segments, loops or a coil-like conformation to bend, fold or laterally displace the formed elongated solid when injected within the tissue space. As described herein, the formed elongated solid has a coiled conformation that is constrained in a straightened conformation when placed in the injector device. Once deployed and unconstrained, the formed elongated solid returns to a coiled conformation in the tissue space. As described herein, the formed elongated solid comprises several separate segments of an elongated solid drug containing material stacked end to end.

The injection of the solid or semi-solid composition may be aided by a lubricant. The lubricant may be coated on the composition, placed in the injection path of the composition or both. The lubricant may include, but is not limited to, a lipid, a fatty acid, polyethylene glycol, a surfactant or oil such as a low molecular weight polysiloxane polymer.

As noted, a variety of drugs may be delivered by the present disclosure to the eye for use in the treatment of a variety of ocular diseases and conditions including inflammation, infection, macular degeneration, retinal degeneration, neovascularization, proliferative vitreoretinopathy, glaucoma, and edema. Useful drugs include, but are not limited to, steroids such as corticosteroids including dexamethasone, fluocinolone, loteprednol, difluprednate, fluorometholone, prednisolone, medrysone, triamcinolone, betamethasone and rimexolone; non- steroidal anti-inflammatory agents such as salicylic-, indole acetic-, aryl acetic-, aryl propionic- and enolic acid derivatives including bromfenac, diclofenac, flurbiprofen, ketorolac tromethamine and nepafenac; antibiotics including azithromycin, bacitracin, besifloxacin, ciprofloxacin, erythromycin, gatifloxacin, gentamicin, levofloxacin, moxifloxacin, ofloxacin, sulfacetamide and tobramycin; VEGF inhibitors such as tyrosine kinase inhibitors, antibodies to VEGF, antibody fragments to VEGF,VEGF binding fusion proteins; PDGF inhibitors, antibodies to PDGF, antibody fragments to PDGF, PDGF binding fusion proteins; anti-TNF alpha agents such as antibodies to TNF-alpha, antibody fragments to TNF-alpha and TNF binding fusion proteins including infliximab, etanercept, adalimumab, certolizumab and golimumab; mTOR inhibitors such as sirolimus, sirolimus analogues, Everolimus, Temsirolimus and mTOR kinase inhibitors; cell therapies such as mesenchymal cells or cells transfected to produce a therapeutic compound; neuroprotective agents such as antioxidants, calcineurin inhibitors, NOS inhibitors, sigma-1 modulators, AMPA antagonists, calcium channel blockers and histone-deacetylases inhibitors; antihypertensive agents such as prostaglandin analogs, beta blockers, alpha agonists, and carbonic anhydrase inhibitors; aminosterols such as squalamine; antihistamines such as H1-receptor antagonists and histamine H2-receptor antagonists; and nucleic acid based therapeutics such as gene vectors, plasmids and siRNA.

In a further description of the disclosure (not according to the invention) there is provided a method of manufacture of the drug composition comprising forming the drug into an elongate body by extrusion, the elongate body comprising a binder or excipient. The drug may be in the form of microspheres. Generally the drug (or drug-containing microspheres) will be mixed with the binder or excipient prior to extrusion into an elongate shape.

In one description of the disclosure, the method of manufacture comprises the steps of:
a) providing a drug composition, optionally in the form of microparticles or microspheres;
b) providing a binder;
c) mixing or combining the drug composition and binder to form a mixture or slurry; and
d) extruding the mixture or slurry to form an elongate body.

The extruded elongate body has a suitable size and shape as described above. The drug composition may be formulated into microspheres with a suitable polymer excipient. The step of mixing or combining the drug composition and binder to form a mixture or slurry may comprise formulation of the drug composition in the binder in a suitable ratio. For example, the mixture or slurry may comprise 5 to 50% binder, for example between 5 to 25% binder.

The step of extrusion generally comprises extruding the mixture of slurry through an appropriately sized needle (for example one having an internal diameter of between 0.25 and 0.6 mm, providing elongate bodies of equal diameter). The method of manufacture may further comprise a step of cutting the extruded mixture or slurry into elongate bodies of lengths suitable for injection into the eye (such as between 1 and 50 mm, for example between 1 and 25 mm).

As described herein, the method may further comprise forming one or more regions of reduced buckling threshold, such as regions of reduced cross-sectional thickness. Such regions may be formed by compressing the elongate body at the desired site or sites of reduced buckling thresholds. This may be achieved after or during extrusion of the elongate body.

In one description of the disclosure (not according to the invention), there is provided the drug composition of the disclosure for use in medicine, in particular for use in ocular medicine. In a further description of the disclosure (not according to the invention), there is provided the drug composition of the disclosure for use in the treatment of an ocular disease or condition. The ocular disease or condition may be inflammation, infection, macular degeneration, retinal degeneration, neovascularization, proliferative vitreoretinopathy, glaucoma or edema. The drug composition is generally for administration by injection, in particular ocular injection.

In another description of the disclosure (not according to the invention) there is provided a kit of parts comprising the injection device described herein and the drug composition of the disclosure. The drug composition may be provided pre-loaded into the device composition. Alternatively, the drug composition may be provided as a plurality of discrete dosage forms suitable for insertion into the delivery device. There is therefore also provided the drug composition of the disclosure in the form of a plurality of discrete dosage forms.

The disclosure will now be described in reference to a number of examples, which are provided for illustrative purposes and are not to be construed as limiting on the scope of the disclosure.

### Examples:

### Reference Example 1. Fabrication of Dexamethasone Containing Microspheres

A microsphere containing dexamethasone was prepared by spray drying a dispersion of polylactic-glycolic acid in a solvent consisting of 90% dichloromethane and 10% methanol. The dispersion was formulated with a solids content of 4.25 % by weight with a drug content of 20% of the solids. The microspheres were collected and washed through a 20 micron filter to remove aggregated material. The resultant drug-containing microspheres were harvested and a sample tested for particle size analysis in a Coulter particle size analyzer. The drug- containing microspheres demonstrated a volumetric mean diameter of 15.12 microns.

### Reference Example 2. Drug Elution of Dexamethasone Containing Microspheres

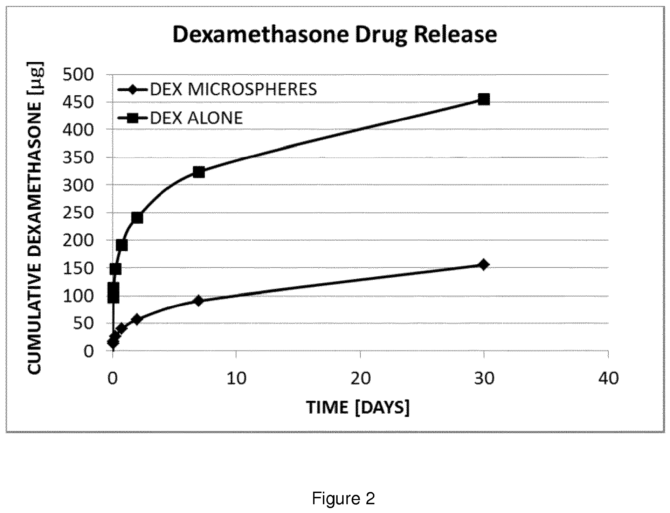

The microspheres of Example 1 were tested for drug release. Samples of the drug- containing microspheres, averaging 5.2 mg, were placed in Eppendorf tubes with 1.2 ml of phosphate buffered saline (PBS) and suspended with agitation. For comparison, samples of the drug used to produce the microspheres, averaging 2.8 mg, were also placed in Eppendorf tubes with 1.2 ml of PBS and suspended with agitation. The tubes were incubated at 37 degrees centigrade with mixing at 250 rpm. At time intervals of 1, 2, 6, 18 and 48 hours a tube was centrifuged and the supernatant removed for drug analysis. For each time point, 10 microliters of supernatant was placed into methanol to solubilize the drug and the resultant drug content was analyzed by HPLC. The HPLC method used a C18 reverse phase column and UV detection and was calibrated to a standard curve of drug. The results indicate that the drug alone had reached saturation of the fluid by 1 hour and at each successive time interval showing an average of 32.7 micrograms of drug per milliliter of fluid. In contrast, the drug-containing microspheres did not reach the saturation concentration demonstrated by the drug alone, demonstrating drug concentration in the range of 3.5 to 12.7 micrograms per milliliter at the 1, 2, 6, 18 and 48 hour time periods. The results were fit in to the Korsmeyer-Peppas equation for drug release from a material with fits of R = 0.998 for the drug-containing microspheres and R = 0.993 for the drug alone. The cumulative drug release profile from the drug-containing microspheres and the drug alone is shown in Fig. 2 from the Korsmeyer-Peppas equation plotted to 30 days.

### Example 3. Fabrication and Injection of Elongated Solid Composition Containing Microspheres

Microspheres fabricated from polylactic-glycolic acid were formulated with polyvinylpyrollidone polyvinylpyrrolidone of 360,000 average molecular weight as a binder. The microspheres were formulated with the polyvinylpyrrolidone in a 10% by weight solution in water. The microspheres comprised 52.1 weight % of the solids content of the formulation with the binder comprising the remaining 47.9 weight %. The formulation was extruded onto a plastic surface through a 27 gauge needle and allowed to dry to produce an elongated formed solid. The resultant dry polymer filaments were cut and sized to fit into the lumen of 25, 27 and 30 gauge needles. A metal plunger was placed in the needle proximal to the solid drug formulation and the solid was delivered from each needle by advancing the plunger.

### Example 4. Fabrication of Elongated Solid Composition Containing Microspheres

Microspheres fabricated from polylactic-glycolic acid were formulated with polyvinylpyrrolidone of 360,000 average molecular weight as a binder. The microspheres were formulated with the polyvinylpyrrolidone in a 10% by weight solution in water. The microspheres comprised 90.8 weight % of the solids content of the formulation with the binder comprising the remaining 9.2 weight %. The formulation was extruded onto a plastic surface through a 27 gauge needle and allowed to dry to produce an elongated formed solid.

### Example 5. Dissolution of Elongated Solid Composition Containing Microspheres

Samples of the elongated formed solid from Examples 3 and 4 were tested for the ability to dissolute in physiological conditions to release microspheres. A 23 mm length filament from Example 3 with weight of 0.67 mg was placed in an Eppendorf tube with 0.5 ml of PBS. A 20 mm length of filament from Example 4 with weight of 1.31 mg was placed in an Eppendorf tube with 0.5 ml of PBS. The tubes were incubated at 37 degrees centigrade in a water bath with a control tube containing only PBS. Fluid samples of the fluid were taken at 5, 10, 20, 30 and 60 minutes of incubation without agitation and inspected with light microscopy at 100 and 200 times magnification. At 5 minutes and subsequent time points, the sample from Example 3 was dissoluted with only free microspheres observed in solution. The sample of Example 4 was partially dissoluted at 5 and 10 minutes with fragments of the sample observed with free microspheres in solution. At 20 and 30 minutes, the sample of Example 4 was observed to consist predominantly of free microspheres in solution, with some aggregates of microspheres and binder. At 60 minutes the sample of Example 4 was observed to consist of free microspheres in solution with occasional aggregates of microspheres. The control sample demonstrated no microspheres at all time periods.

### Example 6. Buckling Strength of Elongated Solid for Injection into the Suprachoroidal Space

An experiment was performed to determine the buckling strength of the elongated formed solid that would not penetrate the choroid of an eye, thereby allowing delivery to the suprachoroidal space. Different suture materials were prepared as mechanical models. Short lengths of suture approximately 2 cm long were cut and the buckling force was measured for each sample. The suture materials and their buckling force is listed in the following table:

| **Suture Size & Type** | **Nominal Diameter (mm)** | **Maximum Buckling Force (mg)** |
|---|---|---|
| 6-0 Polypropylene | 1.0 | 106 |
| 5-0 Polypropylene | 1.5 | 670 |
| 6-0 Nylon Braid | 1.0 | 24 |
| 5-0 Nylon Braid | 1.5 | 50 |
| 5-0 Polyglycolic Acid (PGA) Braid | 1.5 | 180 |
| 4-0 Polyglycolic Acid (PGA) Braid | 2.0 | 202 |

A delivery cannula was fabricated to guide the suture samples under manual force against the choroid to simulate the delivery of an elongated formed solid of the present disclosure through a needle. The delivery cannula consisted of a polytetrafluoroethylene (Teflon^{®}) tube with an ID of 250 microns, equivalent to a 25 gauge hypodermic needle, which was bonded into a plastic Luer hub. The tubing was skived half-way through the diameter, 5 mm from the distal tip, to allow the tubing to be bent at an angle of 90 degrees. This configuration allowed the samples to be loaded into the tube close to the distal end. Samples were loaded into the tube and then advanced using surgical forceps.

A human cadaver eye was prepared for testing. The eye was prepared by first removing Tenon's capsule over the pars plana region. A scleral window was made starting just above the pars plana. A rectangular section of scleral tissue approximately 5 mm x 5 mm was excised from the eye, allowing direct access to the choroid.

Each sample was tested by first loading it into the delivery cannula tube. The distal tip of the tube was held approximately 1 mm from the choroidal surface, perpendicular to the surface and the sample advanced until it contacted the choroid. The samples were then advanced at a near constant rate and observed for any penetration of the choroid. Of the six samples tested, the 5-0 polypropylene suture which had the highest buckling force readily penetrated the choroid. All of the other samples did not penetrate the choroid on repeat trials. In each case where the sample did not penetrate the choroid, the tip of the suture was seen depressing the choroidal tissues until the suture buckled. At the buckling point, the suture folded down against the choroid as it was advanced through the delivery cannula, and a loop of suture would form in the suprachoroidal space. The data indicates that an elongated flexible solid with a buckling force of less than 670 mg of force was suitable for delivery through a cannula to a space such as the suprachoroidal space.

### Reference Example 7. Fabrication of Everolimus Containing Microspheres

A dispersion of polylactic-glycolic acid copolymer and Everolimus in dichloromethane was prepared with a total solids content of 9.3 weight percent. The dispersion was emulsified into a PVA and water solution of 2.5 weight percent, forming a discontinuous phase of polymer and drug microdroplets in a continuous aqueous phase. The emulsion was allowed to mix overnight and the polymer microspheres formed were harvested by filtration and lyophilized to dryness. The resultant microspheres were tested for drug content by extraction into acetonitrile and analysis by reverse phase HPLC. The microspheres demonstrated a drug content of 33.5 weight % of Everolimus. A sample of the microspheres were dispersed in water and analyzed for particle size distribution with a Coulter LS 200 particle size analyzer. The microspheres demonstrated a volumetric mean particle diameter of 17.0 microns.

### Example 8. Fabrication of Elongated Solid Composition Containing Everolimus Microspheres

A formed elongated solid containing drug was fabricated by extruding a slurry of the drug loaded microspheres of Example 7 with an excipient. A slurry for extrusion was formulated using 85 weight % microspheres and 15 weight % polyvinylpyrrolidone. The 15 weight % polyvinylpyrrolidone was formulated from 92 weight % high molecular weight, K90, and 8 weight % low molecular weight, K12, in a solution of 25 weight % concentration in de-ionized water. The slurry was extruded through a 250 micron orifice to create a filament for delivery of size similar to the orifice. The dispersion was dispensed using a 0.3 ml syringe with a distal needle of 0.25 mm inner diameter using a syringe pump at a pump speed setting of 15 microliters/min. Lengths of solid elongated composition in the form of filaments were formed and harvested. The filaments were allowed to dry at ambient conditions prior to further processing. For injection into eyes, the filaments were cut into segments 12 mm in length, corresponding to approximately 0.50 microliters of volume.

### Example 9. Drug Elution of Everolimus Microspheres and Elongated Solid Composition

The microspheres of Example 7 and the elongated solid composition of Example 8 were tested for drug release. Samples of the Everolimus containing microspheres, averaging 4.0 mg, were placed in Eppendorf tubes with 1.2 ml of phosphate buffered saline (PBS) and suspended with agitation. Samples of the elongated solid composition containing Everolimus of 12 mm length, averaging 0.51 mg were placed in Eppendorf tubes with 1.2 ml of phosphate buffered saline (PBS) and suspended with agitation. For comparison, samples of the drug used to produce the microspheres, averaging 4.1 mg, were also placed in Eppendorf tubes with 1.2 ml of PBS and suspended with agitation. The tubes were incubated at 37 degrees centigrade with mixing at 250 rpm. At time intervals of 0, 1, 2, 4 and 7 days a tube containing sample was centrifuged and the supernatant removed for drug analysis. For each time point, 10 microliters of supernatant was placed into 75:25 acetonitrile and water solvent to solubilize the drug and the resultant drug content was analyzed by HPLC. The HPLC method used a C18 reverse phase column and UV detection and was calibrated to a standard curve of drug. The results indicate that Everolimus alone had released 0.45% of the total drug at 7 days. In contrast, the Everolimus containing microspheres released drug at a slower rate, with 0.06% of the drug released at 7 days. Similar to the microspheres, the elongated solid composition containing Everolimus released drug at a slow rate, with 0.04% of the drug released at 7 days.

### Example 10. Injection of Everolimus Containing Elongated Solid Composition

A device was fabricated to inject a solid or semi-solid material into the suprachoroidal or supraciliary space of the eye. A barrel element was fabricated by cutting off the proximal end of a 0.5 ml insulin syringe to a barrel length of 30 mm. The integral needle was removed from the barrel to allow the attachment of standard Luer hub needles. The distal tip of the barrel was cut off leaving a remaining section of Luer taper capable of securely holding a Luer hub needle. A barrel end cap was fabricated from a nylon 10-32 socket head cap screw with a thread length of 4.5 mm. A through hole of 1.86 mm diameter was drilled through the end cap to allow the plunger to freely slide through the end cap. A plunger was fabricated from a tubular stainless steel rod with an outer diameter of 1.8 mm and an inner diameter of 0.8 mm and a length of 43 mm. The distal end of the rod was turned down to a diameter of 1.74 mm and a stainless steel washer of 4.1 mm outer diameter, 1.70 mm inner diameter and 0.5 mm thickness was press-fit onto the rod to provide a distal stop for the plunger spring. The proximal end of the rod was drilled out to 1.55 mm diameter.

A 0.25 mm diameter straightened stainless steel wire 0.25 mm diameter and 80 mm long was used as a push shaft to expel the delivered material from the device. A section of polyetheretherketone (PEEK) capillary tubing with an outer diameter of 1.57 mm, an inner diameter of 0.25 mm and a length of 2.25 mm was used as a securing element for the wire push shaft. The lumen of the PEEK securing element was sufficiently tight enough on the wire push shaft to hold it securely in place under normal use, but allowed the push shaft to be slidably adjusted. The push shaft wire was inserted through the PEEK securing element and then the securing element was press-fit into the drilled out proximal end of the plunger rod with the wire push shaft extending through the lumen of the plunger rod. A compression spring with an outer diameter of 3.1 mm and a wire diameter of 0.18 mm and a length of 31.8 mm was placed over the shaft of the plunger and the barrel end cap was then slid over the plunger shaft proximal to the spring. The plunger and push shaft assembly was placed into the barrel housing with the push shaft extending through the distal tip of the barrel. The end cap was press fit into the barrel proximal end securing the plunger assembly within the barrel.

A 27 gauge by 13 mm hypodermic needle (Nipro Corporation Model AH+2713) was placed over the distal end of the wire push shaft and pressed onto the barrel distal Luer taper. The lumen of the 27 gauge needle allowed for a close sliding fit with the wire push shaft. Once assembled, the length of the push shaft was adjusted so that the tip of the push shaft was at the same level as the distal tip of the 27 gauge needle.

A safety mechanism was incorporated into the device to prevent premature activation of the plunger by the plunger spring force. Two shallow grooves 180 degrees apart and perpendicular to the axis of the plunger were made in the plunger at a distance of 19 mm from the distal tip. The distance between the groove faces was 1.5 mm. A securement clip was fabricated from brass sheet with a width of 6.3 mm and a length of 18 mm. A slot with a width of 1.6 mm and a length of 8.8 mm was machined into the securement clip. The slot was cut in the center of the short side of the securement clip and traversing in the long axis direction.

For use, the plunger was retracted compressing the plunger spring until the plunger grooves were exposed proximally to the end cap. The securement clip was placed over the plunger such that the grooves on the plunger shaft entered the slot on the securement clip. The securement clip then was held against the proximal end surface of the end cap by the spring force, preventing movement of the plunger. The drug containing filament of Example 8 was placed in to the lumen of the 27 gauge needle. A molded cylindrical distal element was fabricated from 70 Shore A durometer silicone rubber. The distal element had a length of 3.7 mm and a diameter of 1.75 mm. The distal element had a lumen of 2.7 mm length and 0.38 mm diameter. The distal end of the lumen of the distal element was configured with a beveled shape which conformed to the bevel on the distal end of a 27 gauge needle. The distal element was attached to the distal tip of the needle such that the needle bevel was in contact with the lumen bevel in order to seal the distal tip of the needle. The non-beveled section of the lumen acted as a slidable seal on the shaft of the needle to provide sufficient frictional force against the needle shaft to maintain the distal tip against the eye surface during advancement of the needle through the distal seal of 1 mm thickness. The distal end of the distal element was fabricated with an end cut at a 60 degree angle to allow for an angled approach to the surface of an eye. The distal sealing element was placed over the distal tip of the needle.

A porcine subject of approximately 24 kg in weight was anesthetized and placed in a prone position. An eye was draped and a speculum inserted to access the eye. Due to the high mobility of the porcine eye, a 6-0 Vicryl suture was placed as a limbal traction suture. The location of the suprachoroidal space was verified by injection of a small amount of air with a thirty gauge needle in the region of the pars plana. The device was positioned with the distal tip on the surface of the eye and the securement clip removed. The needle was advanced through the distal sealing element and into the eye. Once the tip of the needle had reached the suprachoroidal space, the drug containing filament was injected into the space by the device mechanism without triggering of injection by the operator. The procedure was repeated on three other porcine subjects.

After 7 days, two animal subjects were euthanized per protocol. Two eyes treated with the Everolimus containing filament were examined. The injection site showed little or no irritation. The eyes in which the filaments were administered were enucleated for analysis. After 14 days, two animal subjects were euthanized per protocol and two eyes treated with the Everolimus containing filament were examined. The injection site showed little or no irritation. The eyes in which the filaments were administered were enucleated for analysis.

The harvested eyes were frozen and sectioned into anterior and posterior halves at the equator. The posterior portion was dissected to separate the retina and choroidal tissues. The tissues were homogenized in a lysis buffer consisting of 50mM TrisHCl (pH 7.4), 0.15M NaCl, and 0.1% TritonX-100. The resulting homogenate was extracted with acetonitrile, centrifuged and the supernatant filtered with a 0.2 micron filter. The drug content in the filtrate was analyzed by reverse phase HPLC/UV-Vis to determine the drug content of the retinal and choroid tissues. The posterior retina and choroid demonstrated drug concentration of 102.2 ng/mg of tissue after 7 days and 330.3 ng/mg of tissue after 14 days.

### Reference Example 11. Fabrication of Sirolimus Containing Microspheres

A dispersion of polylactic-glycolic acid copolymer and sirolimus in dichloromethane was prepared with a total solids content of 8.4 weight percent. The dispersion was emulsified into a PVA and water solution of 2.5 weight percent, forming a discontinuous phase of polymer and drug microdroplets in a continuous aqueous phase. The emulsion was allowed to mix overnight and the polymer microspheres formed were harvested by filtration and lyophilized to dryness. The resultant microspheres were tested for drug content by extraction into acetonitrile and analysis by reverse phase HPLC. The microspheres demonstrated a drug content of 25 weight % of sirolimus. A sample of the microspheres were dispersed in water and analyzed for particle size distribution with a Coulter LS 200 particle size analyzer. The microspheres demonstrated a volumetric mean particle diameter of 15.7 microns.

### Example 12. Fabrication of Elongated Solid Composition Containing Sirolimus

A formed elongated solid containing drug was fabricated by extruding a slurry of the drug loaded microspheres of example 11 with an excipient. A slurry for extrusion was formulated using 85 weight % microspheres and 15 weight % polyvinylpyrrolidone. The 15 weight % polyvinylpyrrolidone was formulated from 92 weight % high molecular weight, K90, and 8 weight % low molecular weight, K12, in a solution of 25 weight % concentration in de-ionized water. The slurry was extruded through a 250 micron orifice to create a filament for delivery of similar diameter to the orifice. The dispersion was dispensed using a 0.3 ml syringe with a distal needle of 0.25 mm inner diameter using a syringe pump at a pump speed setting of 15 microliters/min. Lengths of solid filaments were formed and harvested. The filament was allowed to dry at ambient conditions prior to further processing. For injection into eyes the filaments were cut into segments 12 mm in length.

### Example 13. Drug elution of Sirolimus Containing Microspheres and Elongated Solid Composition

The microspheres of Example 11 and the elongated solid composition of Example 12 were tested for drug release. Samples of the sirolimus containing microspheres, averaging 4.0 mg, were placed in Eppendorf tubes with 1.2 ml of phosphate buffered saline (PBS) and suspended with agitation. Samples of the elongated solid composition containing sirolimus of 12 mm length, averaging 0.55 mg were placed in Eppendorf tubes with 1.2 ml of phosphate buffered saline (PBS) and suspended with agitation. For comparison, samples of the drug used to produce the microspheres, averaging 3.3 mg, were also placed in Eppendorf tubes with 1.2 ml of PBS and suspended with agitation. The tubes were incubated at 37 degrees centigrade with mixing at 250 rpm. At time intervals of 0, 1, 2, 4 and 7 days a tube containing sample was centrifuged and the supernatant removed for drug analysis. For each time point, 10 microliters of supernatant was placed into 75:25 acetonitrile and water solvent to solubilize the drug and the resultant drug content was analyzed by HPLC. The HPLC method used a C18 reverse phase column and UV detection and was calibrated to a standard curve of drug. The results indicate that sirolimus alone had released drug with an initial burst release, with 4.07 weight % of the drug released immediately at 0 days with the rest of the time points averaging 0.73 weight % of drug. In contrast, the sirolimus containing microspheres released drug progressively without a burst effect, with 0.83 weight % of the drug released at 7 days. Similar to the microspheres, the elongated solid composition containing sirolimus released drug progressively without a burst effect, with 0.1 weight % of the drug released at 7 days. The elongated solid composition provided an additional barrier to drug release to the constituent microspheres. The drug release profile from the microspheres, elongated solid composition and the drug alone is shown in Fig. 4.

### Reference Example 14. Fabrication of Dexamethasone Containing Microspheres

A dispersion of polylactic-glycolic acid copolymer and dexamethasone was prepared in dichloromethane and ethanol solvent. The dispersion was spray dried to form dry microspheres with a mean particle diameter of approximately 15 microns. The resultant microspheres were tested for drug content by extraction into acetonitrile and analysis by reverse phase HPLC. The microspheres demonstrated a drug content of 50 weight % of dexamethasone.

### Example 15. Fabrication of Elongated Solid Composition Containing Dexamethasone Microspheres

A formed elongated solid containing drug was fabricated by extruding a slurry of the drug loaded microspheres of example 14 with an excipient. A slurry for extrusion was formulated using 85 weight % microspheres and 15 weight % polyvinylpyrrolidone. The 15 weight % polyvinylpyrrolidone was formulated from 92 weight % high molecular weight, K90, and 8 weight % low molecular weight, K12, in a solution of 25 weight % concentration in de-ionized water. The slurry was extruded through a 250 micron orifice to create a filament for delivery of similar diameter to the orifice. The dispersion was dispensed using a 0.3 ml syringe with a distal needle of 0.25 mm inner diameter using a syringe pump at a pump speed setting of 15 microliters/min. Lengths of solid filaments were formed and harvested. The filament was allowed to dry at ambient conditions prior to further processing. For injection into eyes, the filaments were cut into segments 12 mm in length.

### Example 16. Drug elution of Dexamethasone Containing Elongated Solid Composition

The elongated solid composition of Example 15 was tested for drug release. Samples of the elongated solid composition containing dexamethasone of 12 mm length, averaging 0.48 mg were placed in Eppendorf tubes with 1.2 ml of phosphate buffered saline (PBS) and suspended with agitation. The tubes were incubated at 37 degrees centigrade with mixing at 250 rpm. At time intervals of 0, 1, 2, 4 and 7 days a tube containing sample was centrifuged and the supernatant removed for drug analysis. For each time point, 10 microliters of supernatant was placed into 50:50 acetonitrile and methanol solvent to solubilize the drug and the resultant drug content was analyzed by HPLC. The HPLC method used a C18 reverse phase column and UV detection and was calibrated to a standard curve of drug. The dexamethasone containing elongated solid composition released drug progressively, with 53.7 % of the drug released at 7 days.

### Example 17. Injection of Dexamethasone Containing Elongated Solid Composition

The drug containing filament of Example 15 was injected into the suprachoroidal space of four porcine subjects as described in Example 10. Two animals were euthanized per protocol after 7 days and two eyes treated with the dexamethasone containing filaments were examined. The injection site showed little or no irritation. The eyes in which a filament was administered were enucleated for analysis. After 14 days, the two animals were euthanized per protocol and two eyes treated with the dexamethasone containing filaments were examined. The injection sites showed little or no irritation. The eyes in which a filament was administered were enucleated for analysis.

The harvested eyes were frozen and sectioned into anterior and posterior halves at the equator. The posterior portion was dissected to separate the retina and choroidal tissues. The tissues were homogenized in a lysis buffer consisting of 50mM TrisHCl (pH 7.4), 0.15M NaCl, and 0.1% TritonX-100. The resulting homogenate was extracted with a solvent containing 50 weight % acetonitrile and 50 weight % methanol, centrifuged and the supernatant filtered with a 0.2 micron filter. The drug content in the filtrate was analyzed by reverse phase HPLC/UV-Vis to determine the drug content of the retinal and choroid tissues. The posterior retina and choroid demonstrated drug concentration of 0.8 ng/mg of tissue after 7 days and 135.0 ng/mg of tissue after 14 days.

### Reference Example 18. Fabrication of Fluocinolone Containing Microspheres

A 6 weight % dispersion of polylactic-glycolic acid copolymer in dichloromethane was prepared and fluocinolone acetonide in ethanol was added to a final solids content of 9.0 weight % in a solvent blend of 94.5 weight % dichloromethane and 4.6 weight % ethanol. The dispersion was emulsified into a PVA and water solution of 2.5 weight percent, forming a discontinuous phase of polymer and drug microdroplets in a continuous aqueous phase. The emulsion was allowed to mix overnight and the polymer microspheres formed were harvested by filtration and lyophilized to dryness. The resultant microspheres were tested for drug content by extraction into acetonitrile and analysis by reverse phase HPLC. The microspheres demonstrated a drug content of 24 weight % of fluocinolone acetonide. A sample of the microspheres were dispersed in water and analyzed for particle size distribution with a Coulter LS 200 particle size analyzer. The microspheres demonstrated a volumetric mean particle diameter of 14.3 microns.

### Example 19. Fabrication of Elongated Solid Composition Containing Fluocinolone

A formed elongated solid containing drug was fabricated by extruding a slurry of the drug loaded microspheres of example 18 with an excipient. A slurry for extrusion was formulated using 85 weight % microspheres and 15 weight % polyvinylpyrrolidone. The 15 weight % polyvinylpyrrolidone was formulated from 92 weight % high molecular weight, K90, and 8 weight % low molecular weight, K12, in a solution of 25 weight % concentration in de-ionized water. The slurry was extruded through a 250 micron orifice to create a filament for delivery with diameter similar to the orifice. The dispersion was dispensed using a 0.3 ml syringe with a distal needle of 0.25 mm inner diameter using a syringe pump at a pump speed setting of 15 microliters/min. Lengths of solid filaments were formed and harvested. The filament was allowed to dry at ambient conditions prior to further processing. For injection into eyes, the filaments were cut into segments 12 mm in length.

### Example 20. Drug elution of Fluocinolone Microspheres and Elongated Solid Composition

The microspheres of Example 18 and the elongated solid composition of Example 19 were tested for drug release. Samples of the fluocinolone containing microspheres, averaging 3.04 mg, were placed in Eppendorf tubes with 1.2 ml of phosphate buffered saline (PBS) and suspended with agitation. Samples of the elongated solid composition containing fluocinolone of 12 mm length, averaging 0.66 mg were placed in Eppendorf tubes with 1.2 ml of phosphate buffered saline (PBS) and suspended with agitation. For comparison, samples of the drug used to produce the microspheres, averaging 4.00 mg, were also placed in Eppendorf tubes with 1.2 ml of PBS and suspended with agitation. The tubes were incubated at 37 degrees centigrade with mixing at 250 rpm. At time intervals of 0, 1, 2, 4 and 7 days a tube containing sample was centrifuged and the supernatant removed for drug analysis. For each time point, 10 microliters of supernatant was placed into 75:25 acetonitrile and water solvent to solubilize the drug and the resultant drug content was analyzed by HPLC. The HPLC method used a C18 reverse phase column and UV detection and was calibrated to a standard curve of drug. The results indicate that fluocinolone alone had released drug with an initial burst release, with 5.3% of the drug released initially at 0 days, followed by an average of 0.9% at subsequent time points. In contrast, the fluocinolone containing microspheres released drug progressively at a slow rate, with 4.7% of the drug released at 7 days. Similar to the microspheres, the elongated solid composition containing fluocinolone released drug progressively at a slow rate, with 6.4% of the drug released at 7 days. The cumulative drug release profile from the fluocinolone containing microspheres and the elongated solid composition containing fluocinolone is shown in Fig. 4.

### Example 21. Injection of Fluocinolone Containing Elongated Solid Composition

The drug containing filament of Example 20 was injected into the suprachoroidal space of four porcine subjects as described in Example 10. After 7 days, two animal subjects were euthanized per protocol and two eyes treated with the fluocinolone containing filaments were examined. The injection site showed little or no irritation. The eyes in which a filament was administered were enucleated for analysis. After 14 days, two animal subjects were euthanized per protocol and two eyes treated with the fluocinolone containing filament were examined. The injection sites showed little or no irritation. The eyes in which a filament was administered were enucleated for analysis.

The harvested eyes were frozen and sectioned into anterior and posterior halves at the equator. The posterior portion was dissected to separate the retina and choroidal tissues. The tissues were homogenized in a lysis buffer consisting of 50mM TrisHCl (pH 7.4), 0.15M NaCl, and 0.1% TritonX-100. The resulting homogenate was extracted with a solvent containing 50 weight % acetonitrile and 50 weight % methanol, centrifuged and the supernatant filtered with a 0.2 micron filter. The drug content in the filtrate was analyzed by reverse phase HPLC/UV-Vis to determine the drug content of the retinal and choroid tissues. The posterior retina and choroid demonstrated drug concentration of 338.8 ng/mg of tissue after 7 days and 1273.3 ng/mg of tissue after 14 days.

### Reference Example 22. Fabrication of Azithromycin Containing Microspheres

A dispersion of polylactic-glycolic acid copolymer and azithromycin in dichloromethane was prepared with a total solids content of 10.0 weight percent with a drug content of 40% of solids. The dispersion was emulsified into a PVA and water solution of 2.5 weight percent, forming a discontinuous phase of polymer and drug microdroplets in a continuous aqueous phase. The emulsion was allowed to mix overnight and the polymer microspheres formed were harvested by filtration and lyophilized to dryness. The resultant microspheres were tested for drug content by extraction into acetonitrile and analysis by reverse phase HPLC. A sample of the microspheres were dispersed in water and analyzed for particle size distribution with a Coulter LS 200 particle size analyzer. The microspheres demonstrated a volumetric mean particle diameter of 22.5 microns.

### Example 23. Fabrication of Elongated Solid Composition Containing Azithromycin

A formed elongated solid containing drug was fabricated by extruding a slurry of the drug loaded microspheres of example 22 with an excipient. A slurry for extrusion was formulated using 85 weight % microspheres and 15 weight % polyvinylpyrrolidone. The 15 weight % polyvinylpyrrolidone was formulated from 92 weight % high molecular weight, K90, and 8 weight % low molecular weight, K12, in a solution of 25 weight % concentration in de-ionized water. The slurry was extruded through a 250 micron orifice to create a filament for delivery of similar diameter to the orifice. The dispersion was dispensed using a 0.3 ml syringe with a distal needle of 0.25 mm inner diameter using a syringe pump at a pump speed setting of 15 microliters/min. Lengths of solid filaments were formed and harvested. The filament was allowed to dry at ambient conditions prior to further processing. For injection into eyes, the filaments were cut into segments 12 mm in length.

### Example 24. Injection of Azithromycin Containing Elongated Solid Composition

A device according to Example 10 was fabricated. The device was prepared for use by retracting the plunger against the spring force and holding it in place with the securement clip. An elongated solid drug composition according to Example 23 was placed in to the lumen of the 27 gauge needle and the molded distal sealing element placed over the distal end of the needle. A cadaver porcine eye was prepared by inflating the posterior chamber to a pressure of approximately 20 mm Hg. A target injection location 5.5 mm posterior of the limbus of the eye was chosen for injection. The securement clip was removed from the plunger shaft. The tissue interface and distal seal was placed against the scleral surface and the needle tip was then advanced through the distal seal and into the tissues. Once the needle lumen reached the suprachoroidal space, the elongated solid drug composition was free to exit the needle and was expelled by the push shaft under the plunger spring force. The delivery of the elongated solid drug composition was confirmed by manually excising a flap in the sclera to expose the suprachoroidal space. A sample of the fluid in the suprachoroidal space was taken and placed on a microscope slide. Examination of the slide under a microscope at 100X magnification revealed numerous microspheres that had been released by the dissolution of the elongated solid drug composition in the suprachoroidal space.

Reference Example 25. Fabrication of Dexamethasone Containing Elongated Solid Composition A formed solid containing drug was fabricated by dissolving polylactic acid polymer in chloroform at a concentration of 16.7 weight %. After the polymer was dispersed in solution a corticosteroid, dexamethasone, was added to the dispersion in a concentration of 60 weight % of the solids content. The dispersion was then used to extrude an elongated solid composition in the form of a filament for use in the device. The dispersion was dispensed using a 0.3 ml syringe with a distal needle of 0.25 mm inner diameter using a syringe pump at a pump speed setting of 50 ul/min. Lengths of solid filaments were formed with diameter ranging from 0.18 mm to 0.25 mm. The filament was allowed to dry and then cut into segments 12 mm in length.

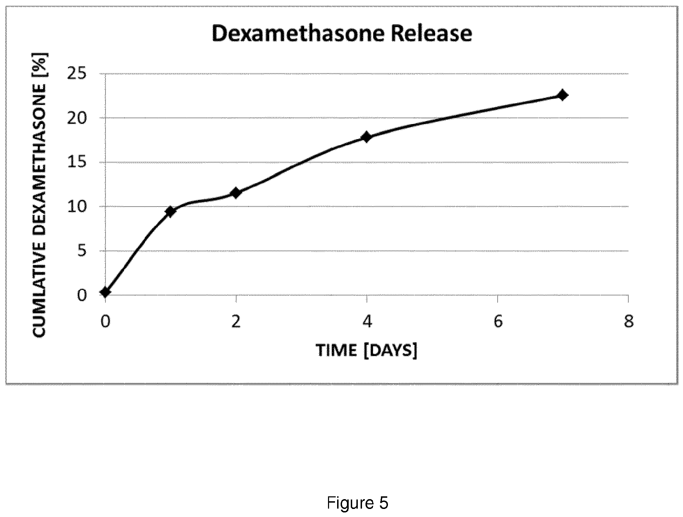

Reference Example 26. Drug elution of Dexamethasone Containing Elongated Solid Composition The elongated solid composition of Example 25 was tested for drug release. Samples of the elongated solid composition containing dexamethasone of 12 mm length, averaging 0.57 mg were placed in Eppendorf tubes with 1.2 ml of phosphate buffered saline (PBS) and suspended with agitation. For comparison, samples of the drug used to produce the microspheres, averaging 2.56 mg, were also placed in Eppendorf tubes with 1.2 ml of PBS and suspended with agitation. The tubes were incubated at 37 degrees centigrade with mixing at 250 rpm. At time intervals of 1, 2, 4 and 7 days a tube containing sample was centrifuged and the supernatant removed for drug analysis. For each time point, 10 microliters of supernatant was placed into 50:50 acetonitrile and methanol solvent to solubilize the drug and the resultant drug content was analyzed by HPLC. The HPLC method used a C18 reverse phase column and UV detection and was calibrated to a standard curve of drug. The dexamethasone containing elongated solid composition drug progressively released drug, with 22.6 weight % of drug released at 7 days. The drug release of the dexamethasone containing elongated solid composition is shown in Fig. 5.

### Reference Example 27. Injection of Dexamethasone Containing Elongated Solid Composition

The drug containing filament of Example 25 was injected into the suprachoroidal space of four porcine subjects as described in Example 10. After 7 days two animals were euthanized per protocol and two eyes treated with the dexamethasone containing filaments were examined. The injection site showed little or no irritation. The eyes in which a filament was administered were enucleated for analysis. After 14 days, two animals were euthanized per protocol and two eyes treated with the dexamethasone containing filament were examined. The injection sites showed little or no irritation. The eyes in which a filament was administered were enucleated for analysis.

The harvested eyes were frozen and sectioned into anterior and posterior halves at the equator. The posterior portion was dissected to separate the retina and choroidal tissues. The tissues were homogenized in a lysis buffer consisting of 50mM TrisHCl (pH 7.4), 0.15M NaCl, and 0.1% TritonX-100. The resulting homogenate was extracted with a solvent containing 50 weight % acetonitrile and 50 weight % methanol, centrifuged and the supernatant filtered with a 0.2 micron filter. The drug content in the filtrate was analyzed by reverse phase HPLC/UV-Vis to determine the drug content of the retinal and choroid tissues. The posterior retina and choroid demonstrated drug concentration of 1.1 ng/mg of tissue after 7 days and 239.3 ng/mg of tissue after 14 days.

### Reference Example 28. Buckling Force Measurement of a Dexamethasone Containing Elongated Solid Composition

An elongated solid composition of Example 25 was tested for the force required to buckle the solid body in the axial direction. A precision load cell (Transducer Techniques Model GSO-50) was mounted to the cross-head of a mechanical tester (Mark-10 Inc, Model ESM301). The output of the transducer was read by a digital strain gauge (Omega Engineering, Model DP-25S). The cross-head was set to a speed of 10 mm/min. Segments of the elongated solid body approximately 3 cm long were placed into a pin vise and the exposed distal length of the segment cut to a gauge length of 2 cm for testing. The pin vise was clamped into a small bench vise and placed on the lower table of the mechanical tester with the distal tip of the elongated solid beneath the compression platen of the load cell. The cross-head was activated and the peak buckling force was recorded from the strain gauge. Five samples were tested, the samples had an average diameter of 0.18 mm and an average buckling force of 634 milligrams.

### Example 29. Elongated Solid Drug Composition with Discrete Regions of Reduced Buckling Threshold or Flexural Rigidity

A device according to Example 10 was fabricated. The solid element filament containing fluocinolone acetonide according to Example 20 was prepared for injection into an eye. To aid in the localized delivery of the solid element in the target space, the microsphere containing filament was cut into segments and loaded into the delivery device. The segments were cut in various decreasing lengths so as allow the length of filament to buckle or move laterally in the suprachoroidal space. The segment lengths from distal to proximal were as follows: 1mm, 2mm, 2mm, 3 mm, and 4 mm for a total length of 12mm.

A cadaver porcine eye was prepared by inflating the posterior chamber to a pressure of approximately 20 mm Hg. A target injection location 5.5 mm posterior of the limbus of the eye was chosen for injection. The securement clip was removed from the plunger shaft. The tissue interface and distal seal was placed against the scleral surface and the needle tip was then advanced through the distal seal and into the tissues. Once the needle lumen reached the suprachoroidal space, the segmented solid element was free to exit the needle and was expelled by the push shaft under the plunger spring force. The delivery of the solid element was confirmed by manually excising a flap in the sclera to expose the suprachoroidal space. A sample of the fluid in the suprachoroidal space was taken and placed on a microscope slide. Examination of the slide under the microscope at 100X magnification revealed numerous microspheres that had been released from the filament injected into the suprachoroidal space.

Example 30. Injection of Elongated Solid Drug Composition into Supraciliary Space of an Eye A Device according to Example 10 was fabricated. A filament solid element according to Example 8 was prepared for injection into an eye.

The device was prepared for use by retracting the plunger against the spring force and holding in place with the securement clip. The filament solid element was placed in to the lumen of the 27 gauge needle. The tissue interface and distal seal was placed over the distal tip of the device needle preventing premature release of the solid element.

A human cadaver eye was prepared by inflating the posterior chamber to a pressure of approximately 15mm Hg. A location 2.5mm posterior of the limbus in the superior-temporal quadrant was marked with a surgical caliper, the location being in the region of the pars plicata, superficial to the ciliary body. The tissue interface of the device was placed against the scleral surface and the safety clip removed. The needle tip was advanced through the distal seal and into the tissues until the needle tip reached the supraciliary space, at which point the plunger advanced the solid element into the space under the force of the plunger spring without manipulation by the operator to trigger injection.

A perfusion bottle of phosphate buffered saline (PBS) was elevated to a height so as to deliver 15mm Hg of fluid pressure to a 30 gauge hypodermic needle. The needle was inserted through the cornea of the cadaver eye, into the anterior chamber and the PBS was allowed to perfuse the eye for 20 hours. After the perfusion, the eye was examined to evaluate the flow of microspheres posteriorly from the injection site. The sclera was carefully dissected away from the ciliary body and choroid and completely removed. The location of the injection could readily be seen as a large, somewhat diffuse concentration of microspheres present on the surface of the choroid and extending in a line posteriorly. Using a glass capillary tube, fluid samples were taken of the choroidal surface in the posterior region of the suprachoroidal space below the implantation site, approximately 8 - 10mm anterior of the optic nerve. The swabs were transferred to a glass microscope slide and examined for the presence of microspheres at 100X magnification. Microspheres were seen in the liquid samples from the posterior region of the eye.

### Reference Example 31: Solid Material Delivery Device

A device according to one description of the disclosure was fabricated to inject a solid material into the suprachoroidal space or supraciliary space of the eye. A body and attached needle was fabricated by cutting off the proximal end of a 0.5 ml insulin syringe with a mm long 27 gauge integral hypodermic needle to a barrel length of 30 mm. The proximal open end of the syringe barrel was tapped for an 8-32 thread. A barrel end cap was fabricated from plastic with a through hole sized to fit the plunger shaft and an external thread of 8-32. A plunger was fabricated from a metal tube with an outer diameter of 1.52 mm and an inner diameter of 0.25 mm. The distal end of the plunger was comprised of two flanges welded to the end with a gap of 1 mm between them. A silicone O-ring seal was placed between the flanges. A compression spring with a 0.20 N/mm spring force, an outer diameter of 2.6 mm and a wire diameter of 0.25 mm was placed over the shaft of the plunger and the barrel end cap was then slid over the plunger shaft proximal to the spring. A solid push shaft of 0.18 mm diameter was fixed in the lumen of the plunger shaft extending distally such that the tip of the push shaft just protruded from the distal tip of the needle when the device was fully assembled and the plunger was in the most distal travel position.

A housing 9.5 mm long with a 1.5 mm outer diameter and a 0.35 mm inner diameter was fabricated from polycarbonate tubing, with a sealing element disposed in the proximal end of the housing. The housing length was such that the distal tip of the housing extended 2 mm beyond the tip of the needle when assembled. A molded tissue interface and distal seal 3.5 mm long with an outer diameter of 1.9 mm and an inner diameter of 0.9 mm was fabricated from 50 Shore A durometer silicone rubber. The tissue interface and distal seal was placed over the distal end of the housing. A housing compression spring of 0.08 N/mm spring force, with an outer diameter of 1.5mm and a wire diameter of 0.1mm was placed over the needle to provide sealing force against the tissues. The housing compression spring had a free length of 4.8 mm and a compressed length of 0.8 mm. The spring was placed over the needle, and then the housing and seal were placed over the needle.

### Reference Example 32: Use of Solid Material Delivery Device

A device according to Example 31 was used to deliver a solid polymer material through a model of the external tissue of the eye to allow visualization of the injection. A hollow spherical article fabricated from silicone elastomer with a durometer of 50 Shore A and a thickness of 1 mm was used to simulate the conjunctiva and sclera of an eye.

The housing, tissue interface and distal seal was temporarily removed from the needle. The plunger and push shaft was retracted proximally and a length of 5-0 polypropylene suture, 9.6 mm in length to act as a solid injection material was inserted into the distal tip of the needle. The polypropylene suture was used as a model for a solid drug containing material. The housing, tissue interface and distal seal were placed back on the needle tip after placement of the suture.

The tissue interface and distal seal of the device was placed against the surface of the model eye and the needle was manually advanced by pushing on the barrel. When the needle had pierced the distal seal and through the model surface tissue, the length of suture was immediately expelled from the needle into the space beneath the model surface tissue without further manipulation of the injection device.

### Reference Example 33: Solid Material Delivery Device

A device according to a description of the disclosure was fabricated to inject a solid or semi-solid material into the suprachoroidal or supraciliary space of the eye. A barrel element was fabricated by cutting off the proximal end of a 0.5 ml insulin syringe to a barrel length of 30 mm. The integral needle was removed from the barrel to allow the attachment of standard Luer hub needles. The distal tip of the barrel was cut off leaving a remaining section of Luer taper capable of securely holding a Luer hub needle. A barrel end cap was fabricated from a nylon 10-32 socket head cap screw with a thread length of 4.5 mm. A through hole of 1.86 mm diameter was drilled through the end cap to allow the plunger to freely slide through the end cap. A plunger was fabricated from a tubular stainless steel rod with an outer diameter of 1.8 mm and an inner diameter of 0.8 mm and a length of 43 mm. The distal end of the rod was turned down to a diameter of 1.74 mm and a stainless steel washer of 4.1 mm outer diameter, 1.70 mm inner diameter and 0.5 mm thickness was press-fit onto the rod to provide a distal stop for the plunger spring. The proximal end of the rod was drilled out to 1.55 mm diameter.

A straightened stainless steel wire 0.25 mm diameter and 80 mm long was used as a push shaft to expel the delivery material from the device. A section of polyetheretherketone (PEEK) capillary tubing with an outer diameter of 1.57 mm, an inner diameter of 0.25 mm and a length of 2.25 mm was used as a securing element for the wire push shaft. The lumen of the PEEK securing element was sufficiently tight enough on the wire push shaft to hold it securely in place under normal use, but allowed the push shaft to be slidably adjusted using moderate force with needle nosed pliers. The push shaft wire was inserted through the PEEK securing element and then the securing element was press-fit into the drilled out proximal end of the plunger rod with the wire push shaft extending through the lumen of the plunger rod. A compression spring with an outer diameter of 3.1 mm and a wire diameter of 0.18 mm and a length of 31.8 mm was placed over the shaft of the plunger and the barrel end cap was then slid over the plunger shaft proximal to the spring. The plunger and push shaft assembly was placed into the barrel housing with the push shaft extending through the distal tip of the barrel. The end cap was press fit into the barrel proximal end securing the plunger assembly within the barrel. A 27 gauge by 13 mm hypodermic needle (Nipro Corporation, Model AH+2713) was placed over the distal end of the wire push shaft and pressed onto the barrel distal Luer taper. The lumen of the 27 gauge needle allowed for a close sliding fit with the wire push shaft. Once assembled, the length of the push shaft was manually adjusted so that the tip of the push shaft was at the same level as the distal tip of the 27 gauge needle.

A safety mechanism was incorporated into the device to prevent premature activation of the plunger by the plunger spring force. Two shallow grooves 180 degrees apart and perpendicular to the axis of the plunger were made in the plunger at a distance of 19 mm from the distal tip. The distance between the groove faces was 1.5 mm. A securement clip was fabricated from brass sheet with a width of 6.3 mm and a length of 18 mm. A slot with a width of 1.6 mm and a length of 8.8 mm was machined into the securement clip. The slot was cut in the center of the short side of the securement clip and traversing in the long axis direction.

For use, the plunger was retracted thereby compressing the plunger spring until the plunger grooves were exposed proximally to the end cap. The securement clip was placed over the plunger such that the slot on the securement clip engaged the grooves on the plunger shaft. The securement clip then was held against the proximal end surface of the end cap by the spring force, preventing movement of the plunger.

### Reference Example 34: Solid Material Delivery Device

A device according to Example 33 was fabricated. A molded cylindrical tissue interface and distal seal element was fabricated from 70 Shore A durometer silicone rubber. The distal element had a length of 3.7 mm and a diameter of 1.75 mm. The distal element had a lumen of 2.7 mm length and 0.38 mm diameter. The distal end of the lumen of the distal element was configured with a beveled shape which conformed to the bevel on the distal end of a 27 gauge needle. The distal element was attached to the distal tip of the needle such that the needle bevel was in contact with the lumen bevel in order to seal the distal tip of the needle. The non-beveled section of the lumen acted as a slidable seal on the shaft of the needle and provided enough frictional force against the needle shaft to maintain the distal tip against the eye surface during advancement of the needle through the distal seal of 1 mm thickness.

### Reference Example 35:

A device according to a description of the disclosure was fabricated to inject a solid or semi-solid material into the subconjunctival space of the eye. A barrel element was fabricated by cutting off the proximal end of a 0.5 ml insulin syringe to a barrel length of 30 mm. The integral needle was removed from the barrel to allow the attachment of a modified needle for subconjunctival injection. The distal tip of the barrel was cut off leaving a remaining section of Luer taper capable of securely holding Luer hub of the needle. A barrel end cap was fabricated from a nylon 10-32 socket head cap screw with a thread length of 4.5 mm. A through hole of 1.86 mm diameter was drilled through the end cap to allow the plunger to freely slide through the end cap. A plunger was fabricated from a tubular stainless steel rod with an outer diameter of 1.8 mm and an inner diameter of 0.8 mm and a length of 43 mm. The distal end of the rod was turned down to a diameter of 1.74 mm and a stainless steel washer of 4.1 mm outer diameter, 1.70 mm inner diameter and 0.5 mm thickness was press-fit onto the rod to provide a distal stop for the plunger spring. The proximal end of the rod was drilled out to 1.55 mm diameter.

A stainless steel wire 0.20 mm diameter and 80 mm long was used as a push shaft to expel the delivery material from the device. A section of polyetheretherketone (PEEK) capillary tubing with an outer diameter of 1.57 mm, an inner diameter of 0.25 mm and a length of 2.25 mm was used as a securing element for the wire push shaft. The push shaft wire was inserted through the PEEK securing element and then the securing element was press-fit into the drilled out proximal end of the plunger rod with the wire push shaft extending through the lumen of the plunger rod. A compression spring with an outer diameter of 3.1 mm and a wire diameter of 0.2 mm and a length of 31.8 mm was placed over the shaft of the plunger and the barrel end cap was then slid over the plunger shaft proximal to the spring. The plunger and push shaft assembly was placed into the barrel housing with the push shaft extending through the distal tip of the barrel. The end cap was press fit into the barrel proximal end securing the plunger assembly within the barrel. A custom 27 gauge by 13 mm hypodermic needle fabricated with an 18 degree bevel angle, short bevel needle was placed over the distal end of the wire push shaft and pressed onto the barrel distal Luer taper. The lumen of the 27 gauge needle allowed for a close sliding fit with the wire push shaft. Once assembled, the length of the push shaft was manually adjusted so that the tip of the push shaft was at the same level as the distal tip of the 27 gauge needle and then the push shaft was adhesively bonded into place at the securing element.

A safety mechanism was incorporated into the device to prevent premature activation of the plunger by the plunger spring force. Two shallow grooves 180 degrees apart and perpendicular to the axis of the plunger were made in the plunger at a distance of 19 mm from the distal tip. The distance between the groove faces was 1.5 mm. A securement clip was fabricated from brass sheet with a width of 6.3 mm and a length of 18 mm. A slot with a width of 1.6 mm and a length of 8.8 mm was machined into the securement clip. The slot was cut in the center of the short side of the securement clip and traversing in the long axis direction.

For use, the plunger was retracted thereby compressing the plunger spring until the plunger grooves were exposed proximally to the end cap. The securement clip was placed over the plunger such that the slot on the securement clip engaged the grooves on the plunger shaft. The securement clip then was held against the proximal end surface of the end cap by the spring force, preventing movement of the plunger.

A molded cylindrical tissue interface and distal seal element was fabricated from 70 Shore A durometer silicone rubber. The distal element had a length of 3.7 mm and a diameter of 1.75 mm. The distal element had a lumen of 2.7 mm length and 0.41 mm diameter. The distal end of the lumen of the distal element was configured with a beveled shape which conformed to the bevel on the distal end of the 27 gauge needle. The distal tip of the element was beveled at a 20 degree angle parallel to the angle in the distal tip of the lumen. The distal element was attached to the distal tip of the needle such that the needle bevel was in contact with the lumen bevel in order to seal the distal tip of the needle. The non-beveled section of the lumen acted as a slidable seal on the shaft of the needle and provided enough frictional force against the needle shaft to maintain the distal tip against the eye surface during advancement of the needle through the distal seal.

### Example 36: Use of Solid Material Delivery Device

A device according to Example 34 was fabricated. A solid element for delivery was fabricated by extruding a slurry comprised of drug loaded microspheres in a carrier material. The drug loaded microspheres comprised polylactic-glycolic acid copolymer spherical particles in the range of 10 to 20 microns in diameter. The microspheres were loaded with 25 weight % fluocinolone acetonide, a corticosteroid. A slurry for extrusion was formulated using 85 weight % microspheres and 15 weight % binder. The binder was formulated from 92 weight % high molecular weight, K90 polyvinylpyrrolidone and 8 weight % low molecular weight, K12 polyvinylpyrrolidone, which was in a solution of 25 weight % concentration in de-ionized water. The slurry was dispensed using a 0.3 ml syringe with a distal needle of 0.25 mm inner diameter at a pump speed of 50 microliters/min using a syringe pump to extrude filaments of similar diameter to the inner diameter of the dispensing needle. The filaments were allowed to dry at ambient conditions prior to further processing. To aid in the localized delivery of the solid element in the target space, the microsphere containing filament was cut into segments and loaded into the delivery device. The segments were cut in various decreasing lengths so as allow the length of filament segments to buckle or move laterally in the suprachoroidal space. The segment lengths from distal to proximal were as follows: 1mm, 2mm, 2mm, 3 mm, and 4 mm for a total length of 12mm.

A cadaver porcine eye was prepared by inflating the posterior chamber to a pressure of approximately 20 mm Hg. A target injection location 5.5 mm posterior of the limbus of the eye was chosen for injection. The securement clip was removed from the plunger shaft. The tissue interface and distal seal was placed against the scleral surface and the needle tip was then advanced through the distal seal and into the tissues. Once the needle lumen reached the suprachoroidal space, the segmented solid element was free to exit the needle and was expelled by the push shaft under the plunger spring force. The delivery of the solid element was confirmed by manually excising a flap in the sclera to expose the suprachoroidal space. A sample of the fluid in the suprachoroidal space was taken and placed on a microscope slide. Examination of the slide under the microscope at 100X magnification revealed numerous microspheres that had been released from the filament injected into the suprachoroidal space.

### Reference Example 37: Use of Solid Material Delivery Device

A device according to Example 34 was fabricated. A solid element for delivery was fabricated by dissolving polylactic acid polymer in chloroform at a concentration of 16.7 weight %. After the polymer was dispersed in solution, a corticosteroid, dexamethasone, was added to the dispersion in a concentration of 60 weight % of the solids content. The dispersion was then used to extrude a filament for use in the device. The dispersion was dispensed using a 0.3 ml syringe with a distal needle of 0.25 mm inner diameter at a pump speed of 50 microliters/min using a syringe pump. The filament had a diameter similar to the inner diameter of the dispensing needle. The filament was allowed to dry and then cut into segments 12 mm in length, corresponding to approximately 0.59 microliters of volume.

The device was prepared for use by retracting the plunger against the spring force and holding it in place with the securement clip. A filament solid element was placed in to the lumen of the 27 gauge needle. A tissue interface and distal sealing element was fabricated similar to Example 35 with the exception that the distal end was cut at a 60 degree angle parallel to the lumen bevel angle to allow for an angled approach to the surface of an eye. The distal element was placed over the distal tip of the needle to seal the needle lumen and prevent premature release of the solid element.

A cadaver porcine eye was prepared by inflating the posterior chamber to a pressure of approximately 20 mm Hg. A target injection location 6 mm posterior of the limbus of the eye was chosen for injection. The securement clip was removed from the plunger shaft. The end of the distal element was placed against the eye and the device was advanced. The needle penetrated the distal seal and entered the tissues of the eye. Once the needle lumen reached the suprachoroidal space, the solid element was free to exit the needle and was expelled by the push shaft under the plunger spring force. The delivery of the solid element was confirmed by excising a flap in the sclera to expose the suprachoroidal space, which revealed the solid filament in the suprachoroidal space.

A live porcine subject was prepared for use of the device loaded with the solid filament. The subject was placed under general anesthesia and the eye stabilized with a traction suture. The location of the suprachoroidal space was verified by injection of a small amount of air with a thirty gauge needle in the region of the pars plana. The device was positioned with the distal tip on the surface of the eye and the securement clip removed. The needle was advanced through the distal sealing element and into the eye. Once the tip of the needle had reached the suprachoroidal space, the drug containing filament was injected into the space.

### Example 38: Use of Solid Material Delivery Device

A Device according to Example 34 was fabricated. A solid element for delivery was fabricated by extruding a slurry comprised of drug loaded microspheres in a carrier material. The drug loaded microspheres comprised polylactic-glycolic acid copolymer spherical particles in the range of 10 to 20 microns in diameter. The microspheres were loaded with 28 weight % Everolimus, a protein kinase inhibitor. A slurry for extrusion was formulated using 85 weight % microspheres and 15 weight % binder material. The binder material was comprised of 89 weight % high molecular weight K90 polyvinylpyrrolidone, 7 weight % low molecular weight K12 polyvinylpyrrolidone and 4 weight % d-alpha tocopheryl polyethylene glucol succinate (Vitamin E-TPGS) which was in a solution of approximately 25 weight % concentration in de-ionized water. The slurry was dispensed using a 0.3 ml syringe with a distal needle of 0.25 mm inner diameter at a pump speed of 15 microliters/min using a syringe pump to extrude filaments of similar diameter to the inner diameter of the dispensing needle. Filaments were allowed to dry at ambient conditions prior to cutting into segments 12 mm long, corresponding to approximately 0.59 microliters of volume.

The device was prepared for use by retracting the plunger against the spring force and holding in place with the securement clip. A filament solid element was placed in to the lumen of the 27 gauge needle. A tissue interface and distal sealing element was fabricated similar to Example 35 with the exception that the distal end was cut at a 60 degree angle parallel to the lumen bevel angle to allow for an angled approach to the surface of an eye. A tissue interface and distal seal was placed over the distal tip of the device needle preventing premature release of the solid element.

A human cadaver eye was prepared by inflating the posterior chamber to a pressure of approximately 15mm Hg. A location 2.5mm posterior of the limbus in the superior-temporal quadrant was marked with a surgical caliper, the location being in the region of the pars plicata, superficial to the ciliary body. The tissue interface of the device was placed against the scleral surface and the safety clip removed. The needle tip was advanced through the distal seal and into the tissues until the needle tip reached the supraciliary space, at which point the plunger advanced the solid element into the space under the force of the plunger spring without manipulation by the operator to trigger injection.

A perfusion bottle of phosphate buffered saline (PBS) was set at a height to deliver 15mm Hg of fluid pressure to a 30 gauge hypodermic needle. The needle was inserted through the cornea of the cadaver eye, into the anterior chamber and the PBS was allowed to perfuse the eye for 20 hours. After the perfusion, the eye was examined to evaluate the flow of microspheres posteriorly from the injection site. The sclera was carefully dissected away from the ciliary body and choroid and completely removed. The location of the injection could readily be seen as a large somewhat diffuse concentration of microspheres present on the surface of the choroid and extending in a line posteriorly. Using a glass capillary tube, fluid samples were taken of the choroidal surface in the posterior region below the implantation site, approximately 8 - 10mm anterior of the optic nerve. The swabs were transferred to a glass microscope slide and examined for the presence of microspheres at 100X magnification. Microspheres were seen in the liquid samples from the posterior region of the eye.

### Reference Example 39:

A device according to one description of the disclosure was fabricated to inject into the suprachoroidal space or supraciliary space of the eye. A body and attached needle was fabricated by cutting off the proximal end of a 1.0 ml insulin syringe with 12.7 mm long 27 gauge integral needle to a barrel length of 32 mm. The proximal open end of the syringe barrel was tapped for a 10-32 thread. A barrel end cap was fabricated from plastic with a through hole sized to fit the plunger shaft and an external thread of 10-32. A plunger was fabricated from a metal tube with an outer diameter of 2.4 mm and an inner diameter of 0.4 mm. The distal end of the plunger comprised two flanges welded to the end and with a gap of 1.3 mm between them. A silicone O-ring seal was placed between the flanges. A compression spring with a spring force of 0.33 N/mm, an outer diameter of 4.6 mm and a wire diameter of 0.4 mm was placed over the shaft of the plunger and the barrel end cap was then slid over the plunger shaft proximal to the spring. The proximal end of the plunger comprised a larger diameter tube sized to allow the insertion of a rubber duck-bill style check valve which was welded to the plunger shaft after assembly of the plunger spring and end cap. The valve was inserted into the larger tube and a female Luer lock fitting was attached over the tube and valve.

A housing 9.5 mm long with a 1.5 mm outer diameter and a 0.35 mm inner diameter, was fabricated from polycarbonate tubing, with a sealing element disposed in the proximal end of the housing. The housing length was such that the distal tip of the housing extended 2 mm beyond the tip of the needle when assembled. A molded tissue interface and distal seal element 3.5 mm long with an outer diameter of 1.9 mm and an inner diameter of 0.9 mm was fabricated from 50 Shore A durometer silicone. The tissue interface and distal seal was placed over the distal end of the housing. A housing compression spring of 0.08 N/mm spring force, an outer diameter of 1.5 mm and a wire diameter of 0.1 mm was placed over the needle to provide sealing force against the tissues. The housing compression spring had a free length of 4.8 mm and a compressed length of 0.8 mm. The spring was placed over the needle, then the housing and tissue interface and distal seal were placed over the needle and the spring was adhesively bonded to the proximal end of the housing at one end and the syringe barrel at the other end.

### Reference Example 40:

A device with a barrel and plunger according to Example 39 was fabricated. A distal housing with a collapsible element was fabricated from stainless steel and nickel-titanium (nitinol) superelastic metal alloy. The housing and collapsible element consisted of proximal and distal stainless steel tubular shafts with two flat, extended segments of nitinol connecting the tubular shafts together. The flat elements formed collapsible elements between the distal and proximal tubular shafts. The proximal shaft was 3.5 mm long and the distal shaft was 2.5 mm long. The tubular shafts were fabricated from two segments of stainless steel tubing, an inner segment of 0.48 mm inner diameter and 0.68 mm outer diameter, and an outer segment of 0.78 mm inner diameter and 1.06 mm outer diameter. The two flat segments of nitinol were placed 180 degrees apart and assembled by press fitting the two tubular shaft segments together, trapping the flat nitinol segments between them. The flat segments were 0.6 mm wide and 0.2 mm thick and after assembly 7.5 mm long. The proximal end of the inner lumen of the distal tubular shaft was sealed by injecting a small amount of 50 Shore A durometer silicone rubber into the lumen and then curing it at 150 degrees C for 10 minutes. A tissue interface and distal seal was fabricated according to Example 37 and was placed over the outside of the distal tubular shaft. The housing assembly was placed over the needle of the device and the needle tip was pushed through the cast inner lumen seal, thereby placing the collapsible element between the tissue interface with distal seal and the body of the device. The two sealing elements effectively sealed the distal tip of the needle within the distal tubular shaft. The collapsible element prevented travel of the distal element distally, but allowed for travel proximally during needle advancement by collapse of the flat segments of nitinol.

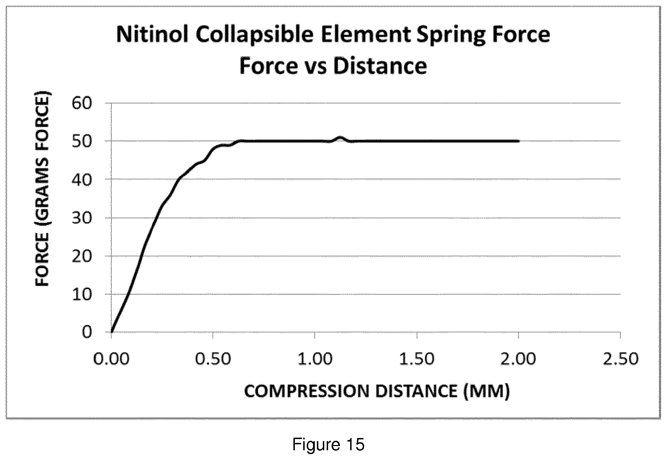

A mechanical testing machine was used to determine the force profile for the distal housing with the collapsible element. An assembled distal housing with the collapsible element was placed over a 27 gauge by 25 mm long needle. The needle was mounted in the lower clamp of the mechanical tester. The upper clamp was configured to allow the needle to slide freely in the clamp and have the jaws of the clamp engage the distal tip of the tissue seal. The mechanical test machine cross-head was set to a compression speed of 25 mm/min. The collapsible distal housing was compressed for a distance of 2.0 mm. Five sample runs were performed and the results were averaged. Fig. 15 presents the averaged results of the force testing of the housing. In the first 0.5 mm of travel the force increased linearly at an average spring rate of 1.07 N/mm. After 0.5 mm of travel the force remained constant at average of 0.49 N.

## Claims

1. A drug composition for delivery to the suprachoroidal space or supraciliary space comprising a flexible solid elongate body for injection into the suprachoroidal space or supraciliary space, wherein the flexible solid elongate body comprises:
(i) a plurality of spherical particles comprising a drug, and
(ii) at least one excipient that acts as a binder to form the spherical particles into the flexible solid,
wherein said spherical particles comprise a biodegradable or bioerodable polymer that undergoes biodegradation or bioerosion in the suprachoroidal space or supraciliary space after injection, wherein said biodegradable or bioerodable polymer is selected from the group consisting of polyhydroxybutyrate, polydioxanone, polyorthoester, polycaprolactone, polycaprolactone copolymers, polycaprolactone-polyethylene glycol copolymers, polylactic acid, polyglycolic acid, polylactic-glycolic acid copolymer and/or polylactic-glycolic acid-ethylene oxide copolymer, and
wherein the binder comprises either (i) a water soluble polymer or (ii) a lipid or fatty acid, wherein said binder undergoes dissolution in the physiological conditions of the suprachoroidal space or supraciliary space after injection to allow the spherical particles to disperse and migrate into the suprachoroidal space or supraciliary space,
wherein the lipid or fatty acid has a melt transition temperature greater than 20 degrees centigrade and up to 37 degrees centigrade.

2. The composition of claim 1 wherein the spherical particles comprise 10% to 90% by weight of the drug.

3. The composition of claim 1 or claim 2 wherein the composition is a flexible solid that comprises approximately 5% to 50% by weight of an excipient that acts as a binder for the drug-containing particles.

4. The composition of claim 3 wherein when the binder comprises a water soluble polymer (i), the water soluble polymer is selected from the group consisting of polyvinylpyrrolidone, polyvinylpyrrolidone co-vinyl acetate, polyvinyl alcohol, polyethylene glycol, polyethylene oxide or chemically modified cellulose.

5. The composition of claim 3 wherein when the binder comprises a lipid or fatty acid (ii), the lipid or fatty acid is selected from the group consisting of capric acid, erucic acid, 1,2-dinervonoyl-sn-glycero-3-phosphocholine, 1,2-dimyristoyl-sn-glycero-3-phosphocholine, or 1,2-dipentadecanoyl-sn-glycero-3-phosphocholine.

6. The composition of any one of claims 1 to 5 wherein the drug is dispersed in the biodegradable or bioerodable material as an amorphous solid dispersion; optionally wherein the drug is dispersed in the biodegradable or bioerodable material as a plurality of drug crystals.

7. The composition of any one of claims 1 to 5 wherein the drug particles additionally comprise a barrier coating.

8. The composition of any one of claims 1 to 7 wherein the flexible elongate body has a buckling force of less than 670 milligrams of force; optionally wherein the flexible solid decreases in buckling force or flexural rigidity when in contact with the suprachoroidal space or supraciliary space.

9. The composition of claim 8 wherein the flexible solid has at least one discrete region of reduced buckling threshold or flexural rigidity along the length of the solid.

10. The composition of claim 9 wherein the discrete region of the flexible solid comprises a region of reduced cross-sectional area; optionally wherein the discrete region of the flexible solid comprises a complete cut across the cross-section.

11. The composition of any one of claims 1 to 10 further comprising a lubricant; optionally wherein the lubricant is a fatty acid, lipid, sterol or oil.

12. The composition of any one of claims 1 to 11 wherein the drug is a steroid, non-steroidal anti-inflammatory agent, anti-TNF alpha agent, mTOR inhibitor, cell therapy, neuroprotective agent or nucleic acid based therapeutic.

13. The composition of claim 12 wherein
(i) the steroid is dexamethasone, fluocinolone, loteprednol, difluprednate, fluorometholone, prednisolone, medrysone, triamcinolone, betamethasone or rimexolone;
(ii) the non-steroidal anti-inflammatory agent is bromfenac, diclofenac, flurbiprofen, ketorolac tromethamine or nepafenac;
(iii) the anti-TNF alpha agent is infliximab, etanercept, adalimumab, certolizumab or golimumab;
(iv) the mTOR inhibitor is sirolimus, Everolimus, Temsirolimus or a mTOR kinase inhibitor;
(v) the cell therapy inhibitor is mesenchymal cells or cells transfected to produce a therapeutic compound;
(vi) the neuroprotective agent is an antioxidant, calcineurin inhibitor, NOS inhibitor, sigma-1 modulator, AMPA antagonist, calcium channel blocker or histone-deacetylases inhibitor; or
(vii) wherein the nucleic acid based therapeutic is a gene vector, plasmid or siRNA.

14. The drug composition of any one of claims 1 to 13 and an injection device comprising:
an elongated barrel with a needle with a lumen at the distal end of the injection device wherein the drug composition of diameter less than or equal to the inner diameter of the needle lumen is contained in the needle lumen;
a plunger with a force element that provides an injection force to the drug composition;
wherein activation of the force element initiates injection of the drug composition.

15. A drug composition of any one of claims 1 to 13 for use in the treatment of an ocular disease or condition by injection to the suprachoroidal space.

16. A drug composition of any one of claims 1 to 13 for use in the treatment of an ocular disease or condition by injection to the supraciliary space.

17. The drug composition for use of claim 15 or claim 16 wherein the drug composition dissolves into a plurality of spherical drug-containing particles; optionally wherein the drug composition dissolves into a plurality of spherical drug-containing particles that migrate in the suprachoroidal space.

18. The drug composition for use of any one of claims 15 to 17 wherein the drug composition is delivered through a needle or cannula.

19. The drug composition for use of any one of claims 15 to 18 wherein the drug composition comprises a steroid, non-steroidal anti- inflammatory agent, antihistamine, aminosterol, antibiotic, VEGF inhibitor, anti-TNF alpha agent, mTOR inhibitor, cell therapy, neuroprotective agent or nucleic acid based therapeutic.

20. The drug composition for use of claim 19 wherein
(i) the steroid is dexamethasone, fluocinolone, loteprednol, difluprednate, fluorometholone, prednisolone, medrysone, triamcinolone, betamethasone or rimexolone;
(ii) the non-steroidal anti-inflammatory agent is bromfenac, diclofenac, flurbiprofen, ketorolac tromethamine or nepafenac;
(iii) the anti-TNF alpha agent is infliximab, etanercept, adalimumab, certolizumab or golimumab;
(iv) the cell therapy inhibitor is mesenchymal cells or cells transfected to produce a therapeutic compound;
(v) the mTOR inhibitor is sirolimus, Everolimus, Temsirolimus or an mTOR kinase inhibitor;
(vi) the neuroprotective agent is an antioxidant, calcineurin inhibitor, NOS inhibitor, sigma-1 modulator, AMPA antagonist, calcium channel blocker or histone-deacetylases inhibitor; or
(vii) the nucleic acid based therapeutic is a gene vector, plasmid or siRNA.

21. The drug composition for use of any one of claims 15 to 20 wherein the ocular disease or condition comprises inflammation, infection, macular degeneration, retinal degeneration, neovascularization, proliferative vitreoretinopathy, glaucoma or edema.

22. The drug composition of claim 1 wherein the spherical particles have an average diameter in the range of 5 to 100 microns, and comprise a mixture of diameters to facilitate close packing.

## Patentansprüche

1. Arzneimittelzusammensetzung zur Abgabe an den suprachoroidalen Raum oder den supraziliären Raum, umfassend einen flexiblen länglichen Feststoffkörper zur Injektion in den suprachoroidalen Raum oder supraziliären Raum, wobei der flexible längliche Feststoffkörper Folgendes umfasst:
(i) eine Mehrzahl sphärischer Teilchen, die ein Arzneimittel umfassen, und
(ii) mindestens einen Hilfsstoff, der als Bindemittel zum Ausbilden der sphärischen Teilchen zu dem flexiblen Feststoff fungiert,
wobei die sphärischen Teilchen ein bioabbaubares oder bioerodierbares Polymer umfassen, das nach der Injektion einen Bioabbau oder eine Bioerosion im suprachoroidalen Raum oder supraziliären Raum durchläuft, wobei das bioabbaubare oder bioerodierbare Polymer ausgewählt ist aus der Gruppe bestehend aus Polyhydroxybutyrat, Polydioxanon, Polyorthoester, Polycaprolacton, Polycaprolacton-Copolymeren, Polycaprolacton-Polyethylenglycol-Copolymeren, Polymilchsäure, Polyglycolsäure, Polymilch-Glycolsäure-Copolymer und/oder Polymilch-Glycolsäure-Ethylenoxid-Copolymer, und
wobei das Bindemittel entweder (i) ein wasserlösliches Polymer oder (ii) ein Lipid oder eine Fettsäure umfasst, wobei das Bindemittel nach der Injektion unter den physiologischen Bedingungen in dem suprachoroidalen Raum oder supraziliären Raum eine Auflösung durchläuft, um zu ermöglichen, dass die sphärischen Teilchen dispergieren und in den suprachoroidalen Raum oder supraziliären Raum wandern,
wobei das Lipid oder die Fettsäure eine Schmelzübergangstemperatur von über 20 Grad Celsius und bis zu 37 Grad Celsius aufweist.

2. Zusammensetzung nach Anspruch 1, wobei die sphärischen Teilchen zu 10 Gew.-% bis 90 Gew.-% das Arzneimittel umfassen.

3. Zusammensetzung nach Anspruch 1 oder Anspruch 2, wobei die Zusammensetzung ein flexibler Feststoff ist, der zu ungefähr 5 Gew.-% bis 50 Gew.-% einen Hilfsstoff umfasst, der als Bindemittel für die Arzneimittel enthaltenden Teilchen fungiert.

4. Zusammensetzung nach Anspruch 3, wobei, wenn das Bindemittel ein wasserlösliches Polymer (i) umfasst, das wasserlösliche Polymer ausgewählt ist aus der Gruppe bestehend aus Polyvinylpyrrolidon, Polyvinylpyrrolidon-Co-Vinylacetat, Polyvinylalkohol, Polyethylenglycol, Polyethylenoxid oder chemisch modifizierter Cellulose.

5. Zusammensetzung nach Anspruch 3, wobei, wenn das Bindemittel ein Lipid oder eine Fettsäure (ii) umfasst, das Lipid oder die Fettsäure ausgewählt ist aus der Gruppe bestehend aus Caprinsäure, Erucasäure, 1,2-Dinervonoyl-sn-glycero-3-phosphocholin, 1,2-Dimyristoyl-sn-glycero-3-phosphocholin oder 1,2-Dipentadecanoyl-sn-glycero-3-phosphocholin.

6. Zusammensetzung nach einem der Ansprüche 1 bis 5, wobei das Arzneimittel in dem bioabbaubaren oder bioerodierbaren Material als amorphe feste Dispersion dispergiert ist; wobei das Arzneimittel gegebenenfalls in dem bioabbaubaren oder bioerodierbaren Material als eine Mehrzahl von Arzneimittelkristallen dispergiert ist.

7. Zusammensetzung nach einem der Ansprüche 1 bis 5, wobei die Arzneimittelteilchen zusätzlich eine Barrierebeschichtung umfassen.

8. Zusammensetzung nach einem der Ansprüche 1 bis 7, wobei der flexible längliche Körper eine Knickkraft von weniger als 670 Milligramm Kraft aufweist; wobei die Knickkraft oder Biegesteifigkeit des flexiblen Feststoffs gegebenenfalls bei Kontakt mit dem suprachoroidalen Raum oder supraziliären Raum abnimmt.

9. Zusammensetzung nach Anspruch 8, wobei der flexible Feststoff mindestens einen separaten Bereich mit verringerter Knickschwelle oder Biegesteifigkeit entlang der Länge des Feststoffs aufweist.

10. Zusammensetzung nach Anspruch 9, wobei der separate Bereich des flexiblen Feststoffs einen Bereich mit verringerter Querschnittsfläche aufweist; wobei der separate Bereich des flexiblen Feststoffs gegebenenfalls einen kompletten Schnitt entlang des Querschnitts umfasst.

11. Zusammensetzung nach einem der Ansprüche 1 bis 10, ferner umfassend ein Gleitmittel; wobei es sich bei dem Gleitmittel gegebenenfalls um eine Fettsäure, ein Lipid, ein Sterol oder Öl handelt.

12. Zusammensetzung nach einem der Ansprüche 1 bis 11, wobei es sich bei dem Arzneimittel um ein Steroid, ein nicht-steroidales entzündungshemmendes Mittel, ein Anti-TNF-alpha-Mittel, einen mTOR-Inhibitor, eine Zelltherapie, ein neuroprotektives Mittel oder ein auf Nukleinsäure basierendes Therapeutikum handelt.

13. Zusammensetzung nach Anspruch 12, wobei
(i) es sich bei dem Steroid um Dexamethason, Fluocinolon, Loteprednol, Difluprednat, Fluormetholon, Prednisolon, Medryson, Triamcinolon, Betamethason oder Rimexolon handelt;
(ii) es sich bei dem nicht-steroidalen entzündungshemmenden Mittel um Bromfenac, Diclofenac, Flurbiprofen, Ketorolactromethamin oder Nepafenac handelt;
(iii) es sich bei dem Anti-TNF-alpha-Mittel um Infliximab, Etanercept, Adalimumab, Certolizumab oder Golimumab handelt;
(iv) es sich bei dem mTOR-Inhibitor um Sirolimus, Everolimus, Temsirolimus oder einen mTOR-Kinase-Inhibitor handelt;
(v) es sich bei dem Zelltherapieinhibitor um mesenchymale Zellen oder um Zellen handelt, die transfiziert werden, um eine therapeutische Verbindung herzustellen;
(vi) es sich bei dem neuroprotektiven Mittel um ein Antioxidans, einen Calcineurin-Inhibitor, NOS-Inhibitor, Sigma-1-Modulator, AMPA-Antagonisten, Calciumkanalblocker oder Histon-Deacetylasen-Inhibitor handelt; oder
(vii) es sich bei dem auf Nukleinsäure basierenden Therapeutikum um einen Genvektor, ein Plasmid oder siRNA handelt.

14. Arzneimittelzusammensetzung nach einem der Ansprüche 1 bis 13 und eine Injektionsvorrichtung, die Folgendes umfasst:
einen länglichen Körper mit einer Nadel mit einem Lumen am distalen Ende der Injektionsvorrichtung, wobei die Arzneimittelzusammensetzung mit einem Durchmesser kleiner oder gleich dem Innendurchmesser des Nadellumens in dem Nadellumen enthalten ist;
einen Kolben mit einem Kraftelement, das der Arzneimittelzusammensetzung eine Injektionskraft bereitstellt;
wobei die Aktivierung des Kraftelements eine Injektion der Arzneimittelzusammensetzung initiiert.

15. Arzneimittelzusammensetzung nach einem der Ansprüche 1 bis 13 zur Verwendung bei der Behandlung einer Augenkrankheit oder eines Augenleidens durch Injektion in den suprachoroidalen Raum.

16. Arzneimittelzusammensetzung nach einem der Ansprüche 1 bis 13 zur Verwendung bei der Behandlung einer Augenkrankheit oder eines Augenleidens durch Injektion in den supraziliären Raum.

17. Arzneimittelzusammensetzung zur Verwendung nach Anspruch 15 oder Anspruch 16, wobei sich die Arzneimittelzusammensetzung zu einer Mehrzahl sphärischer arzneimittelhaltiger Teilchen auflöst; wobei sich die Arzneimittelzusammensetzung gegebenenfalls zu einer Mehrzahl sphärischer arzneimittelhaltiger Teilchen auflöst, die in den suprachoroidalen Raum wandern.

18. Arzneimittelzusammensetzung zur Verwendung nach einem der Ansprüche 15 bis 17, wobei die Arzneimittelzusammensetzung über eine Nadel oder Kanüle abgegeben wird.

19. Arzneimittelzusammensetzung zur Verwendung nach einem der Ansprüche 15 bis 18, wobei es sich bei der Arzneimittelzusammensetzung um ein Steroid, ein nicht-steroidales entzündungshemmendes Mittel, ein Antihistaminikum, ein Aminosterol, ein Antibiotikum, einen VEGF-Inhibitor, ein Anti-TNF-alpha-Mittel, einen mTOR-Inhibitor, eine Zelltherapie, ein neuroprotektives Mittel oder ein auf Nukleinsäure basierendes Therapeutikum handelt.

20. Arzneimittelzusammensetzung zur Verwendung nach Anspruch 19, wobei
(i) es sich bei dem Steroid um Dexamethason, Fluocinolon, Loteprednol, Difluprednat, Fluormetholon, Prednisolon, Medryson, Triamcinolon, Betamethason oder Rimexolon handelt;
(ii) es sich bei dem nicht-steroidalen entzündungshemmenden Mittel um Bromfenac, Diclofenac, Flurbiprofen, Ketorolactromethamin oder Nepafenac handelt;
(iii) es sich bei dem Anti-TNF-alpha-Mittel um Infliximab, Etanercept, Adalimumab, Certolizumab oder Golimumab handelt;
(iv) es sich bei dem Zelltherapieinhibitor um mesenchymale Zellen oder um Zellen handelt, die transfiziert werden, um eine therapeutische Verbindung herzustellen;
(v) es sich bei dem mTOR-Inhibitor um Sirolimus, Everolimus, Temsirolimus oder einen mTOR-Kinase-Inhibitor handelt;
(vi) es sich bei dem neuroprotektiven Mittel um ein Antioxidans, einen Calcineurin-Inhibitor, NOS-Inhibitor, Sigma-1-Modulator, AMPA-Antagonisten, Calciumkanalblocker oder Histon-Deacetylasen-Inhibitor handelt; oder
(vii) es sich bei dem auf Nukleinsäure basierenden Therapeutikum um einen Genvektor, ein Plasmid oder siRNA handelt.

21. Arzneimittelzusammensetzung zur Verwendung nach einem der Ansprüche 15 bis 20, wobei die Augenkrankheit oder das Augenleiden Entzündung, Infektion, Makuladegeneration, Retinadegeneration, Neovaskularisation, proliferative Vitreoretinopathie, Glaukom oder Ödem umfasst.

22. Arzneimittelzusammensetzung nach Anspruch 1, wobei die sphärischen Teilchen einen durchschnittlichen Durchmesser im Bereich von 5 bis 100 Mikrometern aufweisen und eine Mischung von Durchmessern umfassen, um eine dichte Packung zu ermöglichen.

## Revendications

1. Composition pharmaceutique pour administration dans l'espace suprachoroïdien ou l'espace supraciliaire comprenant un corps allongé solide flexible pour injection dans l'espace suprachoroïdien ou l'espace supraciliaire, dans laquelle le corps allongé solide flexible comprend :
(i) une pluralité de particules sphériques comprenant un médicament, et
(ii) au moins un excipient qui agit en tant que liant pour former les particules sphériques en solide flexible,
dans laquelle lesdits particules sphériques comprennent un polymère biodégradable ou bioérodable qui subit une biodégradation ou une bioérosion dans l'espace suprachoroïdien ou l'espace supraciliaire après injection, dans laquelle ledit polymère biodégradable ou bioérodable est choisi dans le groupe constitué des polyhydroxybutyrate, polydioxanone, polyorthoester, polycaprolactone, copolymères de polycaprolactone, copolymères de polycaprolactone-polyéthylène glycol, poly(acide lactique), poly(acide glycolique), copolymère de poly(acide lactique-glycolique) et/ou copolymère de poly(acide lactique-glycolique-oxyde d'éthylène), et
dans laquelle le liant comprend soit (i) un polymère hydrosoluble ou (ii) un lipide ou un acide gras, dans laquelle ledit liant subit une dissolution dans les conditions physiologiques de l'espace suprachoroïdien ou l'espace supraciliaire après injection pour permettre aux particules sphériques de se disperser et de migrer dans l'espace suprachoroïdien ou l'espace supraciliaire,
dans laquelle le lipide ou l'acide gras a une température de transition de fusion supérieure à 20 degrés centigrade et allant jusqu'à 37 degrés centigrade.

2. Composition selon la revendication 1 dans laquelle les particules sphériques comprennent 10 % à 90 % en poids du médicament.

3. Composition selon la revendication 1 ou la revendication 2, la composition étant un solide flexible qui comprend approximativement 5 % à 50 % en poids d'un excipient qui agit en tant que liant pour les particules contenant un médicament.

4. Composition selon la revendication 3 dans laquelle, lorsque le liant comprend un polymère hydrosoluble (i), le polymère hydrosoluble est choisi dans le groupe constitué des polyvinylpyrrolidone, polyvinylpyrrolidone co-acétate de vinyle, alcool polyvinylique, polyéthylène glycol, poly(oxyde d'éthylène) ou cellulose chimiquement modifiée.

5. Composition selon la revendication 3 dans laquelle, lorsque le liant comprend un lipide ou un acide gras (ii), le lipide ou l'acide gras est choisi dans le groupe constitué de l'acide caprique, l'acide érucique, la 1,2-dinervonoyl-sn-glycéro-3-phosphocholine, la 1,2-dimyristoyl-sn-glycéro-3-phosphocholine ou la 1,2-dipentadécanoyl-sn-glycéro-3-phosphocholine.

6. Composition selon l'une quelconque des revendications 1 à 5 dans laquelle le médicament est dispersé dans le matériau biodégradable ou bioérodable sous la forme d'une dispersion solide amorphe ; facultativement dans laquelle le médicament est dispersé dans le matériau biodégradable ou bioérodable sous la forme d'une pluralité de cristaux de médicament.

7. Composition selon l'une quelconque des revendications 1 à 5 dans laquelle les particules de médicament comprennent en outre un enrobage barrière.

8. Composition selon l'une quelconque des revendications 1 à 7 dans laquelle le corps allongé flexible présente une force de gauchissement inférieure à 670 milligrammes de force ; facultativement dans laquelle le solide flexible diminue en termes de force de gauchissement ou de rigidité de flexion lorsqu'il est en contact avec l'espace suprachoroïdien ou l'espace supraciliaire.

9. Composition selon la revendication 8 dans laquelle le solide flexible comporte au moins une région discrète de seuil de gauchissement ou rigidité de flexion réduit le long de la longueur du solide.

10. Composition selon la revendication 9 dans laquelle la région discrète du solide flexible comprend une région d'aire de section transversale réduite ; facultativement dans laquelle la région discrète du solide flexible comprend une coupe complète à travers la section transversale.

11. Composition selon l'une quelconque des revendications 1 à 10 comprenant en outre un lubrifiant ; facultativement dans laquelle le lubrifiant est un acide gras, un lipide, un stérol ou une huile.

12. Composition selon l'une quelconque des revendications 1 à 11 dans laquelle le médicament est un stéroïde, un agent anti-inflammatoire non stéroïdien, un agent anti-TNF alpha, un inhibiteur de mTOR, une thérapie cellulaire, un agent neuroprotecteur ou un agent thérapeutique à base d'acide nucléique.

13. Composition selon la revendication 12 dans laquelle
(i) le stéroïde est la dexaméthasone, la fluocinolone, le lotéprednol, le difluprednate, la fluorométholone, la prednisolone, la médrysone, la triamcinolone, la bétaméthasone ou la rimexolone ;
(ii) l'agent anti-inflammatoire non stéroïdien est le bromfénac, le diclofénac, le flurbiprofène, la kétorolac trométhamine ou le népafénac ;
(iii) l'agent anti-TNF alpha est l'infliximab, l'étanercept, l'adalimumab, le certolizumab ou le golimumab ;
(iv) l'inhibiteur de mTOR est le sirolimus, l'évérolimus, le temsirolimus ou un inhibiteur de mTOR kinase ;
(v) l'inhibiteur de thérapie cellulaire est des cellules mésenchymateuses ou des cellules transfectées pour produire un composé thérapeutique ;
(vi) l'agent neuroprotecteur est un antioxydant, un inhibiteur de calcineurine, un inhibiteur de NOS, un modulateur de sigma-1, un antagoniste d'AMPA, un inhibiteur calcique ou un inhibiteur d'histone-désacétylases ; ou
(vii) dans laquelle l'agent thérapeutique à base d'acide nucléique est un vecteur génique, un plasmide ou un pARNi.

14. Composition pharmaceutique selon l'une quelconque des revendications 1 à 13 et un dispositif d'injection comprenant :
un cylindre allongé avec une aiguille avec une lumière à l'extrémité distale du dispositif d'injection où la composition pharmaceutique de diamètre inférieur ou égal au diamètre interne de la lumière de l'aiguille est confinée dans la lumière de l'aiguille ;
un piston avec un élément de force qui applique une force d'injection à la composition pharmaceutique ;
où l'activation de l'élément de force entraîne l'injection de la composition pharmaceutique.

15. Composition pharmaceutique selon l'une quelconque des revendications 1 à 13 pour utilisation dans le traitement d'une maladie ou affection oculaire par injection dans l'espace suprachoroïdien.

16. Composition pharmaceutique selon l'une quelconque des revendications 1 à 13 pour utilisation dans le traitement d'une maladie ou affection oculaire par injection dans l'espace supraciliaire.

17. Composition pharmaceutique pour utilisation selon la revendication 15 ou la revendication 16 où la composition pharmaceutique se dissout en une pluralité de particules sphériques contenant un médicament ; facultativement où la composition pharmaceutique se dissout en particules sphériques contenant un médicament qui migrent dans l'espace suprachoroïdien.

18. Composition pharmaceutique pour utilisation selon l'une quelconque des revendications 15 à 17 où la composition pharmaceutique est administrée par l'intermédiaire d'une aiguille ou d'une canule.

19. Composition pharmaceutique pour utilisation selon l'une quelconque des revendications 15 à 18 où la composition pharmaceutique comprend un stéroïde, un agent anti-inflammatoire non stéroïdien, un antihistaminique, l'aminostérol, un antibiotique, un inhibiteur de VEGF, un agent anti-TNF alpha, un inhibiteur de mTOR, une thérapie cellulaire, un agent neuroprotecteur ou un agent thérapeutique à base d'acide nucléique.

20. Composition pharmaceutique pour utilisation selon la revendication 19 dans laquelle
(i) le stéroïde est la dexaméthasone, la fluocinolone, le lotéprednol, le difluprednate, la fluorométholone, la prednisolone, la médrysone, la triamcinolone, la bétaméthasone ou la rimexolone ;
(ii) l'agent anti-inflammatoire non stéroïdien est le bromfénac, le diclofénac, le flurbiprofène, la kétorolac trométhamine ou le népafénac ;
(iii) l'agent anti-TNF alpha est l'infliximab, l'étanercept, l'adalimumab, le certolizumab ou le golimumab ;
(iv) l'inhibiteur de thérapie cellulaire est des cellules mésenchymateuses ou des cellules transfectées pour produire un composé thérapeutique ;
(v) l'inhibiteur de mTOR est le sirolimus, l'évérolimus, le temsirolimus ou un inhibiteur de mTOR kinase ;
(vi) l'agent neuroprotecteur est un antioxydant, un inhibiteur de calcineurine, un inhibiteur de NOS, un modulateur de sigma-1, un antagoniste d'AMPA, un inhibiteur calcique ou un inhibiteur d'histone-désacétylases ; ou
(vii) l'agent thérapeutique à base d'acide nucléique est un vecteur génique, un plasmide ou un pARNi.

21. Composition pharmaceutique pour utilisation selon l'une quelconque des revendications 15 à 20 où la maladie ou affection oculaire comprend une inflammation, une infection, la dégénérescence maculaire, la dégénérescence rétinienne, la néovascularisation, la vitréorétinopathie proliférative, le glaucome ou un œdème.

22. Composition pharmaceutique selon la revendication 1 dans laquelle les particules sphériques ont un diamètre moyen dans la plage de 5 à 100 microns, et comprennent un mélange de diamètres pour favoriser le compactage.
